# EUROPEAN PATENT SPECIFICATION

(11) **EP 3 938 368 B1**
(45) Date of publication and mention of the grant of the patent: **07.06.2023**
(21) Application number: 20717027.5
(22) Date of filing: 09.03.2020
(51) Int. Cl.: C07D 487/04

(54) **COMPOUND FORM HAVING ENHANCED BIOAVAILABILITY AND FORMULATIONS THEREOF**
VERBINDUNG FORM MIT VERBESSERTER BIOFÜGBARKEIT UND IHREN FORMULIERUNGEN
FORME D'UN COMPOSÉ AVEC UNE BIOAVAILABILITÉ AMELIORÉE ET SES FORMULATIONS

(30) Priority: 11.03.2019 US 201962816402 P
(43) Date of publication of application: 19.01.2022
(73) Proprietor: PTC Therapeutics, Inc., South Plainfield, NJ 07080 (US)
(72) Inventor: UDDIN, Akm, Nasir, Somerset, NJ 08873 (US); DALI, Mandar, Vasant, Bridgewater, NY 08807 (US); VAZE, Onkar, Shripad, Fords, NJ 08863 (US)
(74) Representative: V.O.
(86) International application number: PCT/US2020/021648
(87) International publication number: WO 2020/185648

(56) References cited:
- WO-A2-2014/081906
- Y. HUANG ET. AL.: "Fundamental aspects of solid dispersion technology for poorly soluble drugs.", ACTA PHARMCEUTICAL SINICA, vol. 4, no. 1, 1 January 2014 (2014-01-01) , pages 18-25, XP055497465, DOI: 10.1016/j.aspb.2013.11.001

## Description

### FIELD

The present description generally relates to amorphous forms of compounds with low aqueous solubility and dissolution rates when in crystalline form (a BCS II class substance), to amorphous solid dispersions of the same, to oral pharmaceutical formulations of the same, and to processes for preparing the amorphous dispersions and formulations. The present description specifically relates to amorphous forms of 5-fluoro-2-(6-fluoro-2-methyl-1H-benzimidazol-1-yl)-N-[4-(trifluoromethyl)phenyl]pyrimidine-4,6-diamine and amorphous solid dispersions and oral pharmaceutical formulations thereof, and to processes for preparing the same.

### BACKGROUND

The bioavailability of an orally administered therapeutic agent is the degree to which the agent is absorbed in the human body and becomes available to an in vivo target (e.g., for interaction or complexation and the like) at a target site (e.g., in or on a cell and the like). To be made bioavailable, a therapeutic agent generally needs to have a certain aqueous solubility with respect to the dose being administered.

The Biopharmaceutics Classification System (BCS) is a system developed by the U.S. Food and Drug Administration (FDA) to differentiate forms of active pharmaceutical ingredients (API) based on their solubility and intestinal permeability, as follows: Class I: high solubility, high permeability; Class II: low solubility, high permeability; Class III: high solubility, low permeability, and Class IV: low solubility, low permeability. Formulating compositions of a BCS II-type API to enhance solubility and thus improve bioavailability can be a challenge.

One of the ways some compounds can be made more soluble is to produce an amorphous form of the compound. However, producing amorphous forms of some compounds can be difficult, and to produce it in a sufficiently stable amorphous form to use in pharmaceutical formulation is particularly difficult. In some instances, an amorphous solid dispersion (ASD) can be used to increase the solubility and bioavailability of some substances. However, identifying an ASD formulation that is stable and does not interfere with the bioavailability of the active pharmaceutical ingredient (API) is difficult.

As a result, there remains a continuing need in the art and a continuing demand in the market for pharmaceutical compositions having improved dissolution and bioavailability that provide ease of dosing and increased API loading.

### SUMMARY

In one aspect described herein, an amorphous form of 5-fluoro-2-(6-fluoro-2-methyl-1H-benzimidazol-1-yl)-N-[4-(trifluoromethyl)phenyl]pyrimidine-4,6-diamine, having the formula of Compound A:

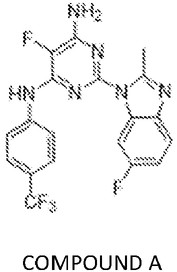

In another aspect described herein, the amorphous form of Compound A is at least 90% amorphous.

In another aspect described herein, the amorphous form of Compound A is a substantially pure amorphous form.

In one aspect described herein, an amorphous solid dispersion (ASD) comprising, the amorphous form of Compound A and a stabilizing polymer.

In another aspect described herein, the stabilizing polymer is selected from polyvinylpyrrolidone or hydroxypropylmethylcellulose acetate succinate.

In another aspect described herein, the stabilizing polymer is hydroxypropylmethylcellulose acetate succinate.

In another aspect described herein, the amount by weight of Compound A to the amount by weight of the stabilizing polymer is in a ratio of between about 5:95 to about 60:40.

In another aspect described herein, the amount by weight of Compound A to the amount by weight of the stabilizing polymer is in a ratio of between about 10:90 to about 40:60.

In one aspect described herein, the ASD is obtained by solvent casting of a solution comprising Compound A and the stabilizing polymer.

In one aspect described herein, the ASD is obtained by spray drying a solution comprising Compound A and the stabilizing polymer.

In one aspect described herein, a process for preparing the ASD comprising, (a) dissolving Compound A and one or more stabilizing polymers in a solvent to form a solution; and, (b) drying the solution to form an ASD, wherein Compound A is present in an amorphous form.

In another aspect described herein, the solvent is selected from acetone, methyl ethyl ketone, dichloromethane, or methanol.

In another aspect described herein, the solution is dried by solvent casting to form the ASD of Compound A.

In another aspect described herein, the solution is dried by spray drying to form the ASD of Compound A.

In one aspect described herein, a pharmaceutical composition comprising, an admixture of the ASD of Compound A and one or more pharmaceutically acceptable excipients.

In another aspect described herein, the one or more pharmaceutically acceptable excipients are selected from a diluent, a disintegrant, a lubricant or a surfactant.

In another aspect described herein, the surfactant is selected from sodium lauryl sulfate or Poloxamer 407.

In one aspect described herein, a process for preparing the pharmaceutical composition comprising, (a) grinding an ASD of Compound A to form a solid dispersion powder; (b) admixing the powder with one or more pharmaceutically acceptable excipients to form an admixture; (c) granulating the admixture to form a granulate; and, (d) tableting or encapsulating the granulate to form tablets or capsules, respectively.

### BRIEF DESCRIPTION OF THE DRAWINGS

FIG. 1 shows a flow diagram showing a process for making tablets described herein from a spray dried dispersion (SDD) of Compound A and a stabilizing polymer.
FIG. 2 shows a power X-ray diffraction profile of raw Compound A (Form I).
FIG. 3 shows an overlay of powder X-ray diffraction profiles of Form I and crystalline polymorph Forms A and B of Compound A.
FIG. 4 shows an overlay of differential scanning calorimetry (DSC) profiles of Forms I, A, and B of Compound A.
FIG. 5 shows a DSC thermograph of HPMC-AS L and HPMC-AS L triturated with Compound A having Form A.
FIG. 6 shows a DSC thermogram of PVP CL and PVP CL triturated with Compound A having Form A.
FIG. 7 shows a DSC thermogram of PVP VA and PVP VA triturated with Compound A having Form A.
FIG. 8 shows a DSC thermogram of PVP K30 and PVP K30 triturated with Compound A having Form A.
FIG 9 shows a DSC thermogram HPMC phthalate and HPMC phthalate triturated with Compound A having Form A.
FIG. 10 shows an X-ray powder diffractogram of Compound A having Form A, a physical mixture of Compound A having Form A and a polymer, and an ASD of HPMCAS-LG based Compound A without a surfactant.
FIG. 11 shows an X-ray powder diffractogram of Compound A having Form A, a physical mixture of Compound A having Form A and a polymer, and an ASD of HPMCAS-LG based Compound A containing Poloxamer 407.
FIG. 12 shows an X-ray powder diffractogram of Compound A having Form A, a physical mixture of Compound A having Form A and a polymer, and an ASD of HPMCAS-LG based Compound A containing sodium lauryl sulphate (SLS).
FIG. 13A shows a DSC thermogram of a physical mixture of Compound A having Form A and a polymer with no surfactant.
FIG. 13B shows a DSC thermogram of an ASD of Compound A containing no surfactant.
FIG. 14A shows a DSC thermogram of a physical mixture of Compound A, polymer, and Poloxamer 407.
FIG. 14B shows a DSC thermogram of an ASD of Compound A containing Poloxamer 407.
FIG. 15A shows a DSC thermogram of a physical mixture of Compound A, polymer, and SLS.
FIG 15B shows a DSC thermogram of an ASD of Compound A containing SLS.
FIG. 16 shows a DSC thermogram of ASDs with different amounts of Compound A.
FIG. 17 shows an X-ray powder diffractogram of ASDs with different amounts of Compound A.
FIG. 18 shows an X-ray powder diffractogram of Compound A and two different SDDs, one with PVP-VA-64 and the other with HPMC-AS-L.
FIG. 19 shows a graph showing dissolution profiles of SDDs of Compound A with HPMC-AS-LG and with PVP-VA-64.
FIG. 20 shows X-ray powder diffractograms of Compound A having Form A, an SDD of Compound A with HPMC-AS, an admixture of Compound A with HPMC-AS used in an active tablet formulation, and HPMC-AS used in a placebo formulation.
FIG. 21 shows the disintegration of Active Formulation Tablets at 1 min with varying concentrations of Poloxamer 407 after storage at 40°C/75% RH under open conditions for 6 months.

### DETAILED DESCRIPTION

In one aspect described herein, an amorphous form of 5-fluoro-2-(6-fluoro-2-methyl-1H-benzimidazol-1-yl)-N-[4-(trifluoromethyl)phenyl]pyrimidine-4,6-diamine, having the formula of Compound A:

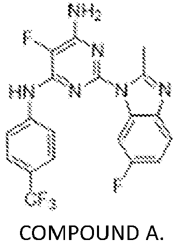

In another aspect described herein, the amorphous form of Compound A is at least 90% amorphous.

In another aspect described herein, the amorphous form of Compound A is a substantially pure amorphous form.

In one aspect described herein, an amorphous solid dispersion (ASD) comprising, the amorphous form of Compound A and a stabilizing polymer.

In another aspect described herein, the stabilizing polymer is selected from polyvinylpyrrolidone and hydroxypropylmethylcellulose acetate succinate.

In another aspect described herein, the stabilizing polymer is hydroxypropylmethylcellulose acetate succinate.

In another aspect described herein, the amount by weight of Compound A to the amount by weight of the stabilizing polymer is in a ratio of between about 5:95 to about 60:40.

In another aspect described herein, the amount by weight of Compound A to the amount by weight of the stabilizing polymer is in a ratio of between about 10:90 to about 40:60.

In one aspect described herein, the ASD is obtained by solvent casting of a solution comprising Compound A and the stabilizing polymer.

In another aspect described herein, the ASD is obtained by spray drying a solution comprising Compound A and the stabilizing polymer.

In one aspect described herein, a process for preparing the ASD comprising, (a) dissolving Compound A and one or more stabilizing polymers in a solvent to form a solution; and, (b) drying the solution to form an ASD, wherein Compound A is present in an amorphous form.

In another aspect described herein, the solvent is selected from acetone, methyl ethyl ketone, dichloromethane, or methanol.

In another aspect described herein, the solution is dried by solvent casting to form the ASD of Compound A.

In another aspect described herein, the solution is dried by spray drying to form the ASD of Compound A.

In one aspect described herein, a pharmaceutical composition comprising, an admixture of the ASD of Compound A and one or more pharmaceutically acceptable excipients.

In another aspect described herein, the one or more pharmaceutically acceptable excipients are selected from a diluent, a disintegrant, a lubricant or a surfactant.

In another aspect described herein, the surfactant is selected from sodium lauryl sulfate or Poloxamer 407.

In one aspect described herein, a process for preparing the pharmaceutical composition comprising, (a) grinding an ASD of Compound A to form a solid dispersion powder; (b) admixing the powder with one or more pharmaceutically acceptable excipients to form an admixture; (c) granulating the admixture to form a granulate; and, (d) tableting or encapsulating the granulate to form tablets or capsules, respectively.

### DEFINITIONS

The term "amorphous," as used herein, refers to a form of a compound in solid state lacking a regular crystalline structure. Without being bound by theory, it is believed that amorphous Compound A requires less energy for dissolution than crystalline Compound A , and this reduced dissolution energy requirement contributes, at least in part, to increased dissolution rate and/or potentially decreased therapeutic onset time exhibited by amorphous form of Compound A and compositions thereof.

The term "Form I" refers to a form of Compound A as described in International Publication Number WO2014/081906. Compound A and methods for making a crystal (non-amorphous) form thereof are disclosed therein. When synthesized according to the process described in WO2014/081906, the Compound A is obtained as a crystalline powder, having a melting point of 240 to 242°C. The compounds disclosed therein are useful in treating cancer, including solid tumors and hematologic cancers such as, but not limited to, diffuse intrinsic pontine glioma (DIPG), ovarian cancer, pancreatic cancer, sarcoma and hematologic cancers, amongst others.

The terms "Form A" and "Form B" refer to forms of Compound A that are purified anhydrous crystalline polymorph forms. Further studies of Compound A, described in Example 1, below, have shown that Form A is the most stable anhydrous crystalline form of the compound, and Form B converts to Form A. In the stable crystalline Form A, Compound A is a BCS II substance.

While oral dosage forms of Compound A in the crystalline Form A may be produced, various methods have been used to increase the solubility and/or dissolution rate of the compound in such formulations, including micronization of Compound A prior to formulation, and the inclusion of pharmaceutical excipients such as disintegrants and surfactants. Examples of such oral dosage forms are provided in Example 2, below.

Solubilized capsule oral dosage forms may be produced from a crystalline form of Compound A. Such capsules may be produced by dissolving Compound A having Form A, in a water miscible organic solvent(s), such as polyethylene glycol 300 or 400, ethanol, propylene glycol, glycerin, N-methyl-2-pyrroloidone, dimethlacetamide, and/or dimethylsulfoxide and a non-ionic surfactant such as polysorbate 20, polysorbate 80, Solutol HS 15, sorbitan monooleate, Poloxamer 407, Gelucire 44/14 or other suitable surfactant. Examples of such capsule formulations are provided in Examples 3 and 4, below. With the inclusion of excipients in the solubilized form, capsule size limited the loading of API in a standard capsule. For instance, the capsule used in Example 4, below, contained 10 or 50 mg of Compound A. In order to administer higher doses of Compound A to a human subject, as was done in the clinical trials discussed in Example 11, more capsules had to be administered to provide the required dose.

### AMORPHOUS FORM OF COMPOUND A

The amorphous form of Compound A disclosed herein is significantly more soluble than the crystalline forms of the compound described above. The amorphous form of Compound A is stable when formulated with a polymer as an amorphous solid dispersion (ASD). The resulting compound-polymer matrix, as discussed below, leads to greater solubility of the amorphous form of Compound A in the ASD and a higher loading capacity of API in the ASD. Accordingly, the amount of the API that can be loaded in a tablet or capsule is significantly increased, thus minimizing the need for the administration of large numbers of tablets or capsules for a single therapeutically effective dose.

In one aspect, the amorphous form of Compound A in the ASD disclosed herein is at least 90% amorphous, more preferably at least 95% amorphous, even more preferably at least 99% amorphous. In another aspect, the amorphous form of Compound A is a substantially pure amorphous form. In another aspect, the substantially pure amorphous form of Compound A contains no detectable amount of a crystalline form of Compound A

### SOLID DISPERSION

The amorphous form of Compound A can be produced in a stable form when combined with a stabilizing polymer in an ASD. As used herein, the term "solid dispersion" refers to an amorphous solid dispersion (ASD), in which an API is present in substantially amorphous form within a stabilizing polymer matrix. To be effective, the stabilizing polymer is preferably compatible with the compound, and capable of releasing the compound from the stabilizing polymer matrix formed therewith when administered to a subject.

Several techniques have been developed to prepare solid dispersions, including hot melt extrusion, co-precipitation, spray drying, hot melt congealing, or solvent casting. The solid dispersions prepared from these different methods may therefore differ in properties based on the matrix formed with the dispersion polymer, such as porosity, surface area, density, stability, hygroscopicity, dissolution and therefore bioavailability.

In one aspect described herein, the ASD of Compound A is produced by solvent casting. In another aspect described herein, the ASD is produced by spray drying, in which case the ASD is in the form of a spray dried dispersion (SDD).

The terms "stabilizing polymer" and "stabilizing polymer matrix" refer to the polymers described herein for preparing an ASD which, when in admixture with an amorphous form of Compound A, form a matrix that is effective in stabilizing the amorphous form of Compound A, thus reducing transformation of amorphous Compound A to crystalline Compound A when contained therein. In one aspect, the stabilizing polymer is selected from hydroxypropylmethylcellulose ("hydromellose" or HPMC), acetate succinate (HPMCAS), polyvinylpyrrolidone ("povidone" or PVP) or a vinylpyrrolidone-vinyl acetate copolymer ("copovidone" or PVP VA 64). The stabilizing polymer is preferably one from which Compound A can be made readily bioavailable to a subject, when orally administered thereto. In another aspect, the stabilizing polymer is selected from HPMCAS, Type L HPMCAS or Type M HPMCAS. Type L refers to a high ratio of succinoyl to acetyl substitution in the polymer while Type M has a medium ratio, each of which dissolve at different pH levels. In another aspect, the stabilizing polymer is Type L HPMCAS.

In one aspect described herein, the ASD can optionally include a surfactant, further enhancing the solubility of Compound A in the ASD. In another aspect, the surfactant is selected from sodium lauryl sulphate and Poloxamer 407. In another aspect, the surfactant is Poloxamer 407.

In one aspect described herein, the ratio of Compound A to stabilizing polymer in the ASD stabilizes the amorphous form of Compound A in the matrix. In another aspect described herein, the compound:polymer ratio is selected from a range of from about 5:95 to about 60:40 by weight, or a range of from about 10:90 to about 40:60 by weight. In another aspect described herein, the compound:polymer ratio is about 40:60 by weight. In one aspect described herein, without excipients present in the ASD, the amount of Compound A loading is selected from a range of from about 30% to about 50% by weight, or a range of from about 30% to about 50% by weight. In another aspect described herein, the amount of Compound A loading is about 40% by weight.

In one aspect described herein, the process for preparing an ASD of Compound A involves dissolving Compound A in one or more stabilizing polymers in a solvent liquid to form a solution, then drying the solution to form a solid dispersion wherein Compound A is present in a compound-polymer matrix as an amorphous form. In another aspect described herein, the drying step is carried out by solvent casting. In another aspect described herein, the drying step is conducted by spray drying. In another aspect described herein, the solvent used is selected based on the solubility of Compound A in the solvent.

In another aspect described herein, the solvent selected conforms to green chemistry requirements and standards as set forth in the Impurities Guidelines for Residual Solvents established by the International Conference on the Harmonization of Technical Requirements for Registration of Pharmaceuticals for Human Uses (ICH Guidelines). In another aspect described herein, mechanical means such as heat and stirring can also be used to facilitate Compound A dissolution in the solvent liquid. The solvent liquid can also comprise a non-organic fraction, for example, water. Non-limiting examples of suitable solvents that may be used include, for example, water-alcohol mixtures, methanol, ethanol, isopropanol, acetone and methyl ethyl ketone. In one aspect, the solvent used is acetone, ethanol, isopropanol or methyl ethyl ketone. In another aspect, the solvent is acetone.

In one aspect described herein, spray drying may be used to produce the ASD of Compound A. Generally, spray drying is a process by which a solution comprising the dissolved substance and a stabilizing polymer is rapidly sprayed over a current of warm air, resulting in formation of dry powder. In another aspect described herein, the ASD may be optionally ground or pulverized to provide a fine or micronized powder form of the ASD. The resulting ASD or powder thereof can be directly filled into capsules for oral administration. In another aspect described herein, the pharmaceutical composition may comprise, the ASD or powder thereof in admixture with one or more additional excipients. In one aspect provided herein, the pharmaceutical composition comprises, an amount of ASD is selected from a range of from about 5 % to about 90% by weight, a range of from about 20% to about 80% by weight, a range of from about 30% to about 70% by weight, or a range of from about 40% to about 60% by weight. In another aspect provided herein, the pharmaceutical composition comprises, an amount of ASD is selected from a range of from 40% to about 60% by weight.

In one aspect provided herein, the ASD or a pharmaceutical composition thereof comprises, an aqueous suspension for oral administration. In another aspect, the aqueous suspension may be prepared by dissolving the ASD or a pharmaceutical composition thereof (e.g., one or more tablets or capsules) in water. In another aspect, a therapeutically effective dose of the aqueous suspension may be administered to a patient, such as a small child, who has difficulty swallowing a tablet or capsule.

### PHARMACEUTICAL COMPOSITIONS

The term "excipient" as used herein means any substance, not itself a therapeutic agent, used as a carrier or vehicle for delivery of a therapeutic agent to a subject or added to a pharmaceutical composition to improve its handling or storage properties or to permit or facilitate formation of the dose unit of the composition into a discrete article, such as a capsule or tablet suitable for oral administration. Excipients include, by way of illustration and not limitation, diluents, disintegrants, binding agents, adhesives, surfactants, lubricants, glidants, surface modifying agents, substances added to mask or counteract a disagreeable taste or odor, flavors, dyes, fragrances, and substances added to improve the appearance of the composition.

In one aspect described herein, a pharmaceutical composition comprising an ASD of Compound A and a stabilizing polymer in admixture with one or more pharmaceutically acceptable excipients may contain a desired amount of Compound A per dose unit and, if intended for oral administration, the pharmaceutical composition can be in the form of a dosage unit selected from a tablet, caplet, pill, hard or soft capsule, a lozenge, a cachet, a powder, granules, or a suspension. In one aspect described herein, the pharmaceutical composition is in the form of a discrete dosage unit containing a predetermined amount of Compound A, such as a tablet or capsule. In a preferred aspect, the discrete dosage unit is a tablet.

### EXCIPIENTS

In another aspect described herein, a pharmaceutical composition described herein may optionally comprise one or more pharmaceutically acceptable diluents as excipients. Suitable diluents, alone or in combination, may be selected from lactose, including anhydrous lactose or lactose monohydrate; starches, including directly compressible and hydrolyzed starches; mannitol; sorbitol; xylitol; dextrose and dextrose monohydrate; sucrose-based diluents including confectioner's sugar; calcium-based diluents including monobasic calcium sulfate monohydrate, dibasic calcium phosphate dihydrate; calcium sulfate dihydrate, or granular calcium lactate trihydrate; dextrans; inositol; hydrolyzed cereal solids; amylose; celluloses including food grade sources of amorphous cellulose and powdered cellulose: microcrystalline cellulose, modified or co-processed microcrystalline cellulose, extragranular microcrystalline cellulose, or silicified microcrystalline cellulose; calcium carbonate; glycine; bentonite; polyvinylpyrrolidone; and the like. In another aspect described herein, such diluents may be present as part of the total weight of the composition in a range selected from about 5% to about 90%, from about 10% to about 60%, or from about 30% to about 50%. The diluent(s) selected preferably exhibit suitable flow properties and, where tablets are desired, improve compressibility.

In another aspect described herein, pharmaceutically acceptable diluents, alone or in combination, may be selected from microcrystalline cellulose, modified or co-processed microcrystalline cellulose, extragranular microcrystalline cellulose, or silicified microcrystalline cellulose or lactose monohydrate. In another aspect described herein, such diluents may be present as part of the total weight of the composition in a range of from about 30% to about 50%. In another aspect described herein, such diluents may be present in about 40% of the total weight of the composition.

In one aspect, lactose and microcrystalline cellulose, either individually or in combination, have been found to be chemically compatible with Compound A after comparison to other diluents. In another aspect, extragranular microcrystalline cellulose, that is microcrystalline cellulose added to a composition after a granulating step, can be used to improve hardness (for tablets). In another aspect, lactose monohydrate provides a pharmaceutical composition having suitable release rates of Compound A, stability, and pre-compression flowability.

In another aspect, pharmaceutical compositions described herein optionally comprise one or more pharmaceutically acceptable disintegrants as excipients, particularly for tablet formulations. Suitable disintegrants include, either individually or in combination, starches, including sodium starch glycolate and pregelatinized corn starches, clays, celluloses such as purified cellulose, microcrystalline cellulose, methylcellulose, carboxymethylcellulose and sodium carboxymethylcellulose, croscarmellose sodium, alginates, crospovidone, and gums such as agar, guar, locust bean, karaya, pectin and tragacanth gums. Croscarmellose sodium is a preferred disintegrant for tablet or capsule disintegration. Croscarmellose sodium confers superior intragranular disintegration capabilities to the granulated compositions described herein.

Disintegrants may be added at any suitable step during the preparation of the composition, particularly prior to granulation or during a lubrication step prior to compression. Such disintegrants, if present, constitute in total about 0.2% to about 30%, preferably about 0.2% to about 10%, more preferably about 1% to about 6% of the total weight of the composition.

Compositions described herein optionally comprise one or more pharmaceutically acceptable binding agents or adhesives as excipients, particularly for tablet formulations. Such binding agents and adhesives preferably impart sufficient cohesion to the powder being tableted to allow for normal processing operations such as sizing, lubrication, compression and packaging, but still allow the tablet to disintegrate and the composition to be absorbed upon ingestion. Suitable binding agents and adhesives include either individually or in combination, acacia; tragacanth; sucrose; gelatin; glucose; starches such as, but not limited to pregelatinized starches; celluloses such as, but not limited to, methylcellulose and carmellose sodium; alginic acid and salts of alginic acid; magnesium aluminum silicate; polyethylene glycol; guar gum; polysaccharide acids; bentonites; povidone, for example povidone K-15, K-30, and K-29/32; polymethacrylates, HPMC, hydroxypropylcellulose; hydroxypropylcellulose acetate succinate (HPMC-AS) and ethylcellulose. Such binding agents and/or adhesives, if present, constitute in total about 0.5% to about 60%, preferably about 0.75% to about 40%, and more preferably about 1% to about 30% of the total weight of the composition.

In one aspect described herein, compositions optionally comprise one or more pharmaceutically acceptable surfactants as excipients to improve the bioavailability of Compound A. Non-limiting examples of surfactants that can be used include quaternary ammonium compounds, for example dioctyl sodium sulfosuccinate, polyoxyethylene alklphenyl ethers, for example nonoxynol 9, nonoxynol 10 and octoxynol 9, Poloxamers (poloxyethylene and polypropylene block copolymers, such as Poloxamer 407), polyoxyethylene fatty acid glycerides and oils, for example polyoxyethylene (8), caprylic/capric mono- and diglycerides, polyoxyethylene (35) castor oil and polyoxyethylene (40) hydrogenated castor oil, polyethylene alkyl ethers, for example poloxyethylene (20) cetostearyl ether, polyoxyethelene fatty acid esters, for example polyoxyethylene (40) stearate, polyoxyethylene sorbitan esters, for example polysorbate 20 and polysorbate 80 (e.g. Tween 80), propylene glycol fatty esters, for example propylene glycol laurate, sodium lauryl sulfate, fatty acids and salts thereof, for example oleic acid, sodium oleate and triethanolamine oleate, glyceryl fatty acid esters, for example sorbitan monolaurate, sorbitan monooleate, sorbitan monopalmitate and sorbitan monostearate, tyloxapol, and mixtures thereof. In another aspect described herein, anionic surfactants such as Poloxamer 407 are particularly preferred. Such surfactants, if present, constitute in total about 0.25% to about 20%, preferably about 0.4% to about 10%, and more preferably about 0.5% to about 5% of the total weight of the composition.

In one aspect described herein, compositions optionally comprise one or more pharmaceutically acceptable lubricants (including anti-adherents and/or glidants) as excipients. Suitable lubricants include, either individually or in combination, glyceryl behaptate; stearic acid and salts thereof, including magnesium, calcium and sodium stearates; hydrogenated vegetable oils; colloidal silica; talc; waxes; boric acid; sodium benzoate; sodium acetate; sodium fumarate; sodium chloride; DL-leucine; polyethylene glycol; sodium oleate; sodium lauryl sulfate; and magnesium lauryl sulfate. In another aspect described herein, magnesium stearate is a preferred lubricant used, for example, to reduce friction between the equipment and granulated mixture during compression of tablet formulations. Such lubricants, if present, constitute about 0.1% to about 10%, preferably about 0.25% to about 5%, more preferably about 0.5% to about 3% of the total weight of the composition.

Suitable anti-adherents include talc, corn starch, DL-leucine, sodium lauryl sulfate and metallic stearates. Talc is a preferred anti-adherent or glidant used, for example, to reduce formulation sticking to equipment surfaces and to reduce static as the composition is mixed. Talc, if present, constitutes about 0.1% to about 10%, more preferably about 0.25% to about 5%, and still more preferably about 0.5% to about 2% of the total weight of the composition.

Glidants can be used to promote powder flow of a solid formulation. Suitable glidants include colloidal silicon dioxide, starch, talc, tribasic calcium phosphate, powdered cellulose and magnesium trisilicate. Colloidal silicon dioxide is particularly preferred. Such glidant, if present, constitutes about 0.1% to about 5%, more preferably about 0.25% to about 5%, more preferably about 0.5% to about 3% of the total weight of the composition.

Other excipients such as colorants, flavors and sweeteners are known in the pharmaceutical art and can be used in compositions described herein. Tablets can be coated, for example with an enteric coating, non-functional cosmetic coating, dry powder compression coating, or uncoated. Compositions described herein can further comprise, for example, buffering agents.

In one aspect described herein, a pharmaceutical composition comprises about 40% to about 60% by weight of an ASD of Compound A and HPMC-AS, preferably a SDD, wherein the ratio of Compound A to HPMC-AS is about 10:90 to about 40:60. In another aspect, the pharmaceutical composition further comprises about 30% to about 50% microcrystalline cellulose and lactose monohydrate, croscarmellose sodium in a range of about 1% to about 6% by weight, Poloxamer 407 in a range of about 0.5% to about 5% by weight, and magnesium stearate in a range of about 0.5% to about 3% by weight. In another aspect, the pharmaceutical composition further comprises colloidal silicon dioxide in a range of about 0.5% to about 2%.

In one aspect described herein, the process for preparing a pharmaceutical composition comprises, admixing an ASD of Compound A admixture with one of more excipients to form an admixture, then tableting or encapsulating the admixture to form tablets or capsules, respectively.

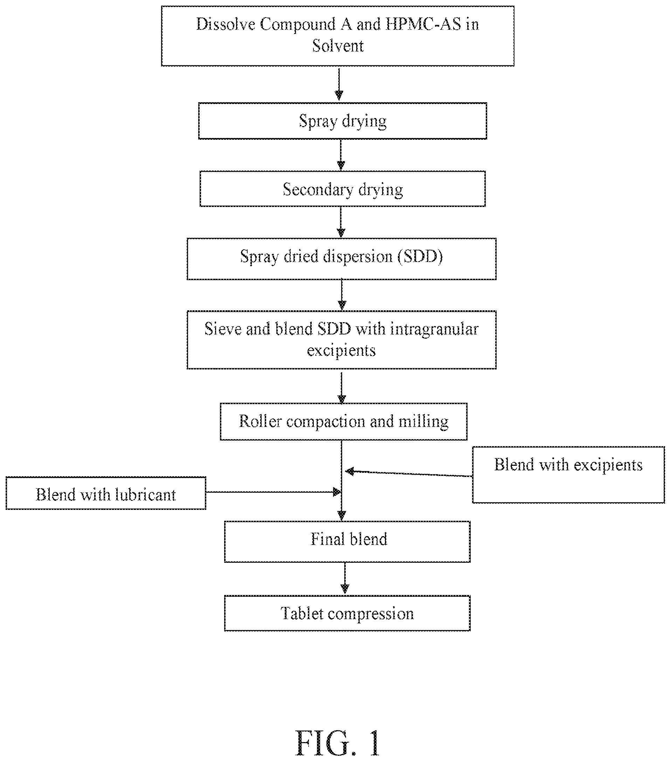

In another aspect described herein, as shown in FIG. 1, the process for preparing a pharmaceutical composition comprises, (a) admixing an ASD of Compound A with one or more excipients to form an admixture, (b) granulating the admixture to form a granulate, and (c) tableting or encapsulating the granulate to form tablets of capsules respectively. The granulate may be prepared initially as a dry granulation. Dry granulation can be accomplished by compaction, for example by roller compaction or slugging, followed by a process, such as crushing in a sieve or shearing in a blender or mill, to form particulate matter. A lubricant is preferably added before tableting. Preparing the granulate can be performed independently under low or high shear. The preferred process forms a granulate that exhibits acceptable content uniformity, readily disintegrates, flows with sufficient ease so that weight variation can be reliably controlled during capsule filling or tableting, and is dense enough in bulk so that a batch can be processed in the selected equipment to exhibit acceptable dosage form characteristics.

Excipients for tablet compositions described herein are preferably selected to provide a disintegration time of less than about 30 minutes, preferably about 20 minutes or less, more preferably about 10 minutes or less, and still more preferably about 5 minutes or less, in a standard disintegration assay.

### THERAPEUTIC DOSES OF COMPOUND A

In one aspect described herein, a pharmaceutical composition includes unit dosage forms of an ASDs of Compound A administered as a dose unit. The term "dose unit" herein means a portion of a pharmaceutical composition that contains an amount of a therapeutic or prophylactic agent, in the present case Compound A, suitable for a single oral administration to provide a therapeutic effect. Typically, one dose unit is desired per single administration to provide a dose comprising a sufficient amount of the agent to result in the desired effect. Administration of such doses can be repeated as required.

As used herein, the terms "effective amount," "prophylactically effective amount" or "therapeutically effective amount" mean an amount of Compound A in the form of an ASD that is effective in producing the desired prophylactic, therapeutic, ameliorative, inhibitory or preventative effect in a cancer in a patient in need thereof.

As used herein, the term "effective amount," in the context of administering a pharmaceutical composition comprising Compound A in the form of an ASD to a patient, refers to the amount of Compound A in the form of an ASD that is sufficient to achieve at least one or more of the following effects, as applicable, in a patient or in patient cell(s): (i) inhibition of tumor proliferation; (ii) reduction in the size or amount of a tumor; (iii) reduction or amelioration in the severity of a cancer or a symptom associated therewith; (iv) prevention of the progression of a cancer or a symptom associated therewith; (v) regression of a cancer or a symptom associated therewith; (vi) prevention of the development or onset of a cancer or a symptom associated therewith; (vii) prevention of the recurrence of a cancer or a symptom associated with the cancer; (viii) reduction of the duration of a symptom associated with a cancer; (ix) reduction or elimination of the cancer stem cell or tumor stem cell population; (x) reduction or elimination of the growth of a tumor or neoplasm; (xi) reduction or elimination of the proliferation of cancer cells or tumor cells; (xii) reduction or elimination of the formation of a tumor or neoplasm overexpressing ; (xiii) eradication or control of a primary, regional and/or metastatic cancer; (xiv) reduction in patient mortality; (xv) increased number of patients in remission; (xvi) increased length of remission in patients; (xvii) the size of a tumor or neoplasm is maintained or controlled such that the size does not increase or increases less than the size of the tumor after administration of a standard therapy as measured by conventional methods available to one of skill in the art, such as MRI, X-ray and CAT scan; (xviii) increased delay in disease progression; (xix) increased patient survival; (xx) reduction in incidences of patient hospitalization; (xxi) reduction in the length of patient hospitalization; (xxii) enhancement or improvement in the prophylactic or therapeutic effect(s) of another therapy; (xxiii) reduction in the number of symptoms associated with a cancer; (xxiv) increased cancer-free survival of patients; and/or (xxv) increased symptom-free survival of cancer patients.

In general, the term "effective amount" also includes that amount of Compound A in the form of an ASD administered to a patient which is in a range of from about 0.001 mg/Kg/day to about 500 mg/Kg/day, or about 0.01 mg/Kg/day to about 500 mg/Kg/day, or about 0.1 mg to about 500 mg/Kg/day, or about 1.0 mg/day to about 500 mg/Kg/day, in single, divided, or a continuous dose for a patient or subject having a weight in a range of between about 40 to about 200 Kg (which dose may be adjusted for patients or subjects above or below this range, particularly children under 40 Kg). The typical adult subject is expected to have a median weight in a range of between about 60 to about 100 Kg. The effective amount for the subject will also depend upon various factors, including the body weight, size and health of the subject. An effective amount for a given patient can be determined according to the skill and judgment of the clinician.

In another aspect, where daily doses are adjusted based upon the weight of the subject or patient, Compound A in the form of an ASD may be formulated for delivery at about 2, 5, 10, 20, 50, 80, 100, 150, 200, 250, 300 or 500 mg/Kg/day. Daily doses adjusted based upon the weight of the subject or patient may be administered as a single, divided, or continuous dose. In another aspect, where a dose of Compound A in the form of an ASD is given more than once per day, the dose may be administered once, twice, three times, or more per day. In another aspect, a subject is administered one or more doses of an effective amount of Compound A in the form of an ASD, wherein the effective amount may not be the same for each dose. Another aspect described herein includes an effective amount of Compound A in the form of an ASD in a range of from about 0.001 mg/Kg/day to about 500 mg/Kg/day.

Within the scope described herein, the "effective amount" of Compound A in the form of an ASD for use in the manufacture of a medicament or in a method for treating a cancer in a subject in need thereof, is intended to include an amount in a range of from about 0.1 ng to about 3500 mg administered daily; from about 0.1 µg to about 3500 mg administered daily; from about 0.1 mg to about 3500 mg administered daily; from about 1 mg to about 3500 mg administered daily; from about 1 mg to about 3000 mg administered daily; from about 0.05 mg to about 1500 mg administered daily; from about 0.5 mg to about 1500 mg administered daily; from about 1 mg to about 1500 mg administered daily; from about 5 mg to about 1500 mg administered daily; from about 10 mg to about 600 mg administered daily; from about 0.5 mg to about 2000 mg administered daily; or, an amount in a range of from about 5.0 mg to about 1500 mg administered daily.

Another aspect described herein includes an effective amount of Compound A in the form of an ASD in a range of from about 0.1 ng to about 3500 mg.

In one aspect described herein, the pharmaceutical composition is a tablet or capsule administered in a therapeutically effective dose in a range of from about 10 mg to about 1000 mg, or from about 50 mg to about 400 mg, or from about 50 mg to about 200 mg. In another aspect described herein, a typical dose unit contains about 10, 20, 25, 50, 75, 100, 125, 150, 175, 200, 250, 300, 350 or 400 mg of Compound A as an ASD. In one aspect, for an adult human, a therapeutically effective amount of Compound A in the form of an ASD per dose unit in a composition described herein is typically about 50 mg to about 400 mg. In another aspect, a therapeutically effective amount of Compound A in the form of an ASD per dose unit in a composition described herein is about 100 mg to about 200 mg, for example about 100 mg or about 200 mg. In another aspect, a therapeutically effective amount of Compound A in the form of an ASD per dose unit as described herein is either about 50 mg or about 200 mg. A dose unit containing a therapeutically effective amount of Compound A in the form of an ASD can be selected to accommodate the desired frequency of administration used to achieve a desired daily dosage.

In one or more aspects described herein, an effective amount for a composition of Compound A in the form of an ASD to be administered for any type of cancer can be estimated initially by *in vitro* results from cell culture assays using patient cells or cell lines known to those skilled in the art or by *in vivo* results from relevant animal models, such as the mouse, chimpanzee, marmoset or tamarin animal model. Relevant animal models may also be used to determine the appropriate concentration range and route of administration. Such information can then be used to determine useful doses and routes for administration in humans. Therapeutic efficacy and toxicity may be determined by standard pharmaceutical procedures in cell cultures or experimental animals, e.g., ED₅₀ (the dose therapeutically effective in 50% of the population) and LD₅₀ (the dose lethal to 50% of the population). The dose ratio between the toxic and therapeutic effect is referred to as the therapeutic index, and can be expressed as the ratio, LD₅₀/ED₅₀. In some aspects, the effective amount is such that a large therapeutic index is achieved. In further aspects, the dosage is within a range of plasma concentrations that include an ED₅₀ with little or no toxicity. The dosage may vary within this range depending upon the dosage form employed, sensitivity of the patient, and the route of administration.

In one aspect described herein, the concentration-biological effect (pharmacodynamic) relationship observed with regard to Compound A in the form of an ASD suggests a target plasma concentration ranging from about 0.001 µg/mL to about 50 µg/mL, from about 0.01 µg/mL to about 20 µg/mL, from about 0.05 µg/mL to about 10 µg/mL, or from about 0.1 µg/mL to about 5 µg/mL. In another aspect described herein, the target plasma concentration may be in a range of from about 3 hr. µg/mL to about 70 hr- µg/mL, from about 3 hr- µg/mL to about 60 hr- µg/mL, from about 3 hr- µg/mL to about 50 hr- µg/mL, from about 3 hr- µg/mL to about 40 hr- µg/mL, from about 3 hr- µg/mL to about 30 hr- µg/mL, from about 3 hr- µg/mL to about 20 hr- µg/mL, from about 3 hr- µg/mL to about 10 hr. µg/mL, and the like, or any range in between.

To achieve such plasma concentrations, Compound A in the form of an ASD may be administered at doses that vary from 0.001 µg to 100,000 mg, depending upon the route of administration in single, divided, or continuous doses for a patient weighing between about 40 to about 100 kg (which dose may be adjusted for patients above or below this weight range, particularly for children under 40 kg).

The exact dosage will be determined by the practitioner, in light of factors related to the subject. Dosage and administration may be adjusted to provide sufficient levels of the active agent(s) or to maintain the desired effect. Administration factors that may be taken into account include the severity of the disease state, general health of the subject, ethnicity, age, weight, and gender of the subject, diet, time and frequency of administration, drug combination(s), reaction sensitivities, tolerance for toxicity related to drug metabolites, experience with other cancer therapies and regimens, and tolerance/response to such therapies and regimens. Long-acting pharmaceutical compositions may be administered every 2, 3 or 4 days, once every week, or once every two weeks depending on half-life and clearance rate of the particular formulation.

Compound A in the form of an ASD may be administered to the subject via oral, ocular, rectal, buccal, topical, nasal, ophthalmic, subcutaneous, intramuscular, intravenous (bolus and infusion), intracerebral, transdermal, and pulmonary routes of administration.

The term "oral administration" herein includes any form of delivery of a therapeutic agent or a composition thereof to a subject wherein the agent or composition is placed in the mouth of the subject, whether or not the agent or composition is immediately swallowed. Absorption of the agent can occur in any part or parts of the gastrointestinal tract including the mouth, esophagus, stomach, duodenum, ileum and colon. The term "orally deliverable" herein means suitable for oral administration.

### USE OF ASD COMPOSITIONS

Pharmaceutical compositions of Compound A in the form of an amorphous solid dispersion (ASD), including any of the dosage forms disclosed herein, can be used in the treatment of various types of cancer, including solid tumors and hematologic cancers, by administering a therapeutically effective amount of an oral dosage form of the composition to a subject in need thereof. The dosage form administered preferably comprises a solid dispersion of Compound A, as disclosed herein.

One aspect described herein includes non-limiting examples of the types of cancer that can be treated with the pharmaceutical compositions of Compound A in the form of an amorphous solid dispersion disclosed herein such as: leukemias, such as but not limited to, acute leukemia, acute lymphocytic leukemia, acute myelocytic leukemias, such as, myeloblastic, promyelocytic, myelomonocytic, monocytic, and erythroleukemia leukemias and myelodysplastic syndrome; chronic leukemias, such as but not limited to, chronic myelocytic (granulocytic) leukemia, chronic lymphocytic leukemia, hairy cell leukemia; polycythemia vera; lymphomas such as but not limited to Hodgkin's lymphoma, non-Hodgkin's lymphoma; multiple myelomas such as but not limited to smoldering multiple myeloma, nonsecretory myeloma, osteosclerotic myeloma, plasma cell leukemia, solitary placancercytoma and extramedullary placancercytoma; Waldenström's macroglobulinemia; monoclonal gammopathy of undetermined significance; benign monoclonal gammopathy; heavy chain disease; bone and connective tissue sarcomas such as but not limited to bone sarcoma, osteosarcoma, chondrosarcoma, Ewing's sarcoma, malignant giant cell tumor, fibrosarcoma of bone, chordoma, periosteal sarcoma, soft-tissue sarcomas, angiosarcoma (hemangiosarcoma), fibrosarcoma, Kaposi's sarcoma, leiomyosarcoma, liposarcoma, lymphangiosarcoma, neurilemmoma, rhabdomyosarcoma, synovial sarcoma; glial brain tumors (i.e., gliomas) such as but not limited to, astrocytoma, ependymoma, oligodendroglioma, brain stem glioma, optic glioma, diffuse intrinsic pontine glioma, mixed glioma (i.e., oligoastrocytoma), glioblastoma, glioblastoma multiforme, nonglial tumor, acoustic neurinoma, craniopharyngioma, medulloblastoma, meningioma, pineocytoma, pineoblastoma, primary brain lymphoma; breast cancer including but not limited to ductal carcinoma, adenocarcinoma, lobular (cancer cell) carcinoma, intraductal carcinoma, medullary breast cancer, mucinous breast cancer, tubular breast cancer, papillary breast cancer, Paget's disease, and inflammatory breast cancer; adrenal cancer such as but not limited to pheochromocytoma and adrenocortical carcinoma; thyroid cancer such as but not limited to papillary or follicular thyroid cancer, medullary thyroid cancer and anaplastic thyroid cancer; pancreatic cancer such as but not limited to, insulinoma, gastrinoma, glucagonoma, VIPoma, somatostatin-secreting tumor, and carcinoid or islet cell tumor; pituitary cancers such as but limited to Cushing's disease, prolactin-secreting tumor, acromegaly, and diabetes insipidus; eye cancers such as but not limited to ocular melanoma such as iris melanoma, choroidal melanoma, and ciliary body melanoma, and retinoblastoma; vaginal cancers such as squamous cell carcinoma, adenocarcinoma, and melanoma; vulvar cancer such as squamous cell carcinoma, melanoma, adenocarcinoma, basal cell carcinoma, sarcoma, and Paget's disease; cervical cancers such as but not limited to, squamous cell carcinoma, and adenocarcinoma; uterine cancers such as but not limited to endometrial carcinoma and uterine sarcoma; ovarian cancers such as but not limited to, ovarian epithelial carcinoma, borderline tumor, germ cell tumor, and stromal tumor; esophageal cancers such as but not limited to, squamous cancer, adenocarcinoma, adenoid cystic carcinoma, mucoepidermoid carcinoma, adenosquamous carcinoma, sarcoma, melanoma, placancercytoma, verrucous carcinoma, and oat cell (cancer cell) carcinoma; stomach cancers such as but not limited to, adenocarcinoma, fungating (polypoid), ulcerating, superficial spreading, diffusely spreading, malignant lymphoma, liposarcoma, fibrosarcoma, and carcinosarcoma; colon cancers; rectal cancers; liver cancers such as but not limited to hepatocellular carcinoma and hepatoblastoma; gallbladder cancers such as adenocarcinoma; cholangiocarcinomas such as but not limited to papillary, nodular, and diffuse; lung cancers such as non-small cell lung cancer, squamous cell carcinoma (epidermoid carcinoma), adenocarcinoma, large-cell carcinoma and small-cell lung cancer; testicular cancers such as but not limited to germinal tumor, seminoma, anaplastic, classic (typical), spermatocytic nonseminoma, embryonal carcinoma, teratoma carcinoma, choriocarcinoma (yolk-sac tumor), prostate cancers such as but not limited to, prostatic intraepithelial neoplasia, adenocarcinoma, leiomyosarcoma, and rhabdomyosarcoma; penal cancers; oral cancers such as but not limited to squamous cell carcinoma; basal cancers; salivary gland cancers such as but not limited to adenocarcinoma, mucoepidermoid carcinoma, and adenoid cystic carcinoma; pharynx cancers such as but not limited to squamous cell cancer, and verrucous; skin cancers such as but not limited to, basal cell carcinoma, squamous cell carcinoma and melanoma, superficial spreading melanoma, nodular melanoma, lentigo malignant melanoma, acral lentiginous melanoma; kidney cancers such as but not limited to renal cell carcinoma, adenocarcinoma, hypernephroma, fibrosarcoma, transitional cell cancer (renal pelvis and/ or ureter); Wilms' tumor; bladder cancers such as but not limited to transitional cell carcinoma, squamous cell cancer, adenocarcinoma, carcinosarcoma. In addition, cancers include myxosarcoma, osteogenic sarcoma, endotheliosarcoma, lymphangioendothelioma, mesothelioma, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas (for a review of such disorders, see Fishman et al., 1985, Medicine, 2d Ed., J.B. Lippincott Co., Philadelphia and Murphy et al., 1997, Informed Decisions: The Complete Book of Cancer Diagnosis, Treatment, and Recovery, Viking Penguin, Penguin Books U.S.A., Inc., United States of America).

Another aspect described herein includes additional examples of the types of cancer that can be treated with the pharmaceutical compositions of Compound A in the form of an ASD disclosed herein such as: a carcinoma, including that of the bladder, breast, colon, kidney, liver, lung, ovary, pancreas, stomach, cervix, thyroid and skin; including squamous cell carcinoma; hematopoietic tumors of lymphoid lineage, including leukemia, acute lymphocytic leukemia, acute lymphoblastic leukemia, B-cell lymphoma, T cell lymphoma, Burkitt's lymphoma; hematopoietic tumors of myeloid lineage, including acute and chronic myelogenous leukemias and promyelocytic leukemia; tumors of mesenchymal origin, including fibrosarcoma and rhabdomyosarcoma; other tumors, including melanoma, seminoma, teratocarcinoma, neuroblastoma; tumors of the central and peripheral nervous system, including astrocytoma, neuroblastoma, glioma, and Schwannomas; tumors of mesenchymal origin, including fibrosarcoma, rhabdomyosarcoma, and osteosarcoma; and other tumors, including melanoma, xeroderma pigmentosum, keratoacanthoma, seminoma, thyroid follicular cancer and teratocarcinoma.

Another aspect described herein includes additional examples of the types of cancer that can be treated with the pharmaceutical compositions of Compound A in the form of an ASD disclosed herein such as: a cancer associated with aberrations in apoptosis including, but not limited to: follicular lymphomas, carcinomas with p53 mutations, hormone dependent tumors of the breast, prostate and ovary, and precancerous lesions such as familial adenomatous polyposis, and myelodysplastic syndromes. Another aspect described herein includes additional examples of the types of cancer that can be treated with the pharmaceutical compositions of Compound A in the form of an ASD disclosed herein such as: a malignancy or dysproliferative changes (such as metaplasia and dysplasia), or hyperproliferative disorders of the skin, lung, liver, bone, brain, stomach, colon, breast, prostate, bladder, kidney, pancreas, ovary, and/or uterus are treated in accordance with the methods described herein. Another aspect described herein includes additional examples of the types of cancer that can be treated with the pharmaceutical compositions of Compound A in the form of an ASD disclosed herein such as: a sarcoma or melanoma.

In a specific aspect, the cancer being treated as described herein is leukemia, lymphoma or myeloma (e.g., multiple myeloma). Non-limiting examples of leukemias and other blood-borne cancers that can be treated with the methods described herein include acute lymphoblastic leukemia (ALL), acute lymphoblastic B-cell leukemia, acute lymphoblastic T-cell leukemia, acute myeloblastic leukemia (AML), acute promyelocytic leukemia (APL), acute monoblastic leukemia, acute erythroleukemic leukemia, acute megakaryoblastic leukemia, acute myelomonocytic leukemia, acute nonlymphocytic leukemia, acute undifferentiated leukemia, chronic myelocytic leukemia (CML), chronic lymphocytic leukemia (CLL), and hairy cell leukemia.

Non-limiting examples of lymphomas that can be treated in accordance with the methods described herein include Hodgkin's lymphoma, non-Hodgkin's lymphoma, multiple myeloma, Waldenström's macroglobulinemia, heavy chain disease, and polycythemia vera.

In another aspect, the cancer being treated as described herein is a solid tumor. Examples of solid tumors that can be treated in accordance with the methods described herein include, but are not limited to fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophageal cancer, stomach cancer, oral cancer, nasal cancer, throat cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular cancer, small cell lung carcinoma, bladder carcinoma, lung cancer, epithelial carcinoma, glioma, glioblastoma multiforme, astrocytoma, medulloblastoma, craniopharyngioma, ependymoma, pinealoma, hemangioblastoma, acoustic neuroma, oligodendroglioma, meningioma, skin cancer, melanoma, neuroblastoma, and retinoblastoma.

In another aspect, the cancer being treated as described herein includes, but is not limited to, brain cancer, gastric cancer, hematologic cancer, lung cancer, non-small cell lung cancer, pancreatic cancer, prostate cancer, salivary gland cancer, colorectal carcinoma, hepatocellular carcinoma, liver carcinoma, breast carcinomas or sarcomas, esophageal carcinomas or sarcomas, stomach carcinomas or sarcomas, fibrosarcoma, glioblastoma, diffuse intrinsic pontine glioma, medulloblastoma, neuroblastoma, diffuse large B cell lymphomas, B cell non-Hodgkin's lymphoma, Hodgkin's lymphoma or chronic or acute myeloid leukemia.

In another aspect, the cancer being treated as described herein includes, but is not limited to, tumors that relapse after therapy despite improved surgical and irradiation techniques. Tumor relapse may occur for a number of reasons, with one plausible explanation being the existence of cancer stem cells (CSC) or tumor stem cells (tumor initiating cells) in the tumor population. CSCs are defined as a population of stem cells relative to any type of blood cancer, solid tumor cancer or metastatic cancer. Tumor stem cells are those specifically found within a tumor. Both have characteristics similar to normal stem cells. Like normal stem cells, CSCs and tumor stem cells have the potential to self-renew. Unlike normal stem cells, though, CSCs and tumor stem cells fail to terminally differentiate and proliferate unchecked. Their enhanced DNA repair capacity also enables them to become resistant to cytotoxic, chemotherapeutic drugs designed to kill cancer cells and tumor cells. Therefore, targeting CSCs and tumor stem cells could be an approach for effective cancer treatment. One further approach is to target various transcription factors responsible for maintenance of the self-renewal capacity of CSCs and tumor stem cells.

As used herein, the term "treat," "treatment" or "treating" refers to: (i) preventing a disease, disorder and/or condition from occurring in a subject that may be predisposed to the disease, disorder and/or condition but has not yet been diagnosed as having said disease, disorder and/or condition; (ii) inhibiting a disease, disorder and/or condition, i.e., arresting its development; and/or (iii) relieving a disease, disorder and/or condition, i.e., causing regression of the disease, disorder and/or condition.

As used herein, the term "subject" refers to members of the human, equine, porcine, bovine, murine, rattus, canine and feline species. In some aspects, the subject is a mammal or a warm-blooded vertebrate animal. In other aspects, the subject is a human. As used herein, the term "patient" may be used interchangeably with "subject" and "human".

In certain aspects, the subject is a human that is 0 to 6 months old, 6 to 12 months old, 6 to 18 months old, 18 to 36 months old, 1 to 5 years old, 5 to 10 years old, 10 to 15 years old, 15 to 20 years old, 20 to 25 years old, 25 to 30 years old, 30 to 35 years old, 35 to 40 years old, 40 to 45 years old, 45 to 50 years old, 50 to 55 years old, 55 to 60 years old, 60 to 65 years old, 65 to 70 years old, 70 to 75 years old, 75 to 80 years old, 80 to 85 years old, 85 to 90 years old, 90 to 95 years old or 95 to 100 years old. In some aspects, the subject is a human infant. In other aspects, the subject is a human toddler. In other aspects, the subject is a human child. In other aspects, the subject is human adult. In yet other aspects, the subject is an elderly human.

As used herein, the term "elderly human" refers to a human 65 years or older; the term "human adult" refers to a human that is 18 years or older; the term "human child" refers to a human that is 1 year to 18 years old; the term "human infant" refers to a newborn to 1 year old year human; and, the term "human toddler" refers to a human that is 1 year to 3 years old.

In certain aspects, the subject is in an immunocompromised state or immunosuppressed state or at risk for becoming immunocompromised or immunosuppressed. In certain aspects, the subject is receiving or recovering from an immunosuppressive therapy. In certain aspects, the subject has or is at risk of getting cancer, AIDS, or a bacterial infection. In certain aspects, the subject is, will or has undergone surgery, chemotherapy and/or radiation therapy. In certain aspects, the subject has cystic fibrosis, pulmonary fibrosis or another condition affecting the lungs. In certain aspects, the subject has, will have or had a tissue transplant.

In some aspects, a cancer may have become refractory to treatment by conventional "standard of care" therapies such that the patient has discontinued the conventional therapy. In one aspect, without being limited by theory, the term "refractory" means that at least some significant portion of the cancer cells, tumor cells, cancer stem cells or tumor stem cells continue to proliferate despite therapy. The determination of whether the cancer is refractory toa particular therapy can be made either in vivo or in vitro by any method known in the art for assaying the effect of a therapy on the cancer cells, tumor cells, cancer stem cells or tumor stem cells, using the art-accepted meanings of "refractory" in such a context. In certain aspects, a patient having a refractory cancer is a patient in which the cancer is non-responsive or resistant to a conventional or "standard of care" therapy. In certain aspects, a patient with refractory cancer has a cancer that progresses. Disease progression, as a lack of clinical response to a therapy, is demonstrated when the tumor or neoplasm has not been significantly eradicated and/or the symptoms have not been significantly alleviated. The determination of whether a patient has a refractory cancer can be made either in vivo or in vitro by any method known in the art for assaying the effectiveness of the therapy for the treatment of the cancer, using art-accepted meanings of "refractory" in such a context.

In certain aspects, the patient to be treated in accordance with the methods described herein is a patient already being treated with antibiotics, anti-virals, anti-fungals, or other biological therapy, immunotherapy or anti-cancer therapy. Among these patients are patients with a refractory cancer or patients too young for conventional therapies. In some aspects, the patient being treated is treatment naive, not having received any prior therapy. In any of the foregoing aspects, a patient to be treated may receive a small molecule therapy.

In some aspects, an ASD form of Compound A may be prophylactically administered to a patient to prevent the onset of a cancer in a patient at risk of developing cancer. In some aspects, an ASD form of Compound A may be therapeutically administered to a patient that is susceptible to adverse reactions to conventional therapies. In some aspects, the subject being administered an ASD form of Compound A has not received prior therapy. In other aspects, an ASD form of Compound A is administered to a subject who has received a prior therapy. In some aspects, the subject administered an ASD form of Compound A has discontinued a prior therapy due to lack of benefit from the therapy, adverse effects from the therapy or unacceptable levels of toxicity.

In some aspects, the subject being administered an ASD form of Compound A will or has undergone surgery, chemotherapy, antibody therapy, hormonal therapy and/or radiation therapy. In certain aspects, the patient has undergone surgery to remove the tumor or neoplasm. In certain aspects, the subject will have, or has had, or is undergoing a tissue or organ transplant.

For any composition of Compound A, the effective amount to be administered for any type of cancer can be estimated initially by *in vitro* results from cell culture assays using patient cells or cell lines known to those skilled in the art or by *in vivo* results from relevant animal models, such as the mouse, chimpanzee, marmoset or tamarin animal model. Relevant animal models may also be used to determine the appropriate concentration range and route of administration. Such information can then be used to determine useful doses and routes for administration in humans. Therapeutic efficacy and toxicity may be determined by standard pharmaceutical procedures in cell cultures or experimental animals, e.g., ED₅₀ (the dose therapeutically effective in 50% of the population) and LD₅₀ (the dose lethal to 50% of the population). The dose ratio between the toxic and therapeutic effect is referred to as the therapeutic index, and can be expressed as the ratio, LD₅₀/ED₅₀. In some aspects, the effective amount is such that a large therapeutic index is achieved. In further aspects, the dosage is within a range of plasma concentrations that include an ED₅₀ with little or no toxicity. The dosage may vary within this range depending upon the dosage form employed, sensitivity of the patient, and the route of administration.

More specifically, the concentration-biological effect (pharmacodynamic) relationship observed with regard to Compound A suggests a target plasma concentration ranging from about from approximately 3 hr- µg/mL to approximately 70 hr. µg/mL, from approximately 3 hr·µg/mL to approximately 60 hr- µg/mL, from approximately 3 hr- µg/mL to approximately 50 hr·µg/mL, from approximately 3 hr- µg/mL to approximately 40 hr- µg/mL, from approximately 3 hr·µg/mL to approximately 30 hr- µg/mL, from approximately 3 hr- µg/mL to approximately 20 hr·µg/mL, from approximately 3 hr- µg/mL to approximately 10 hr·µg/mL, and the like, or any range in between. To achieve such plasma concentrations, Compound A or a form thereof described herein may be administered at doses that vary from 0.001 µg to 100,000 mg, depending upon the route of administration in single, divided, or continuous doses for a patient weighing between about 40 to about 100 kg (which dose may be adjusted for patients above or below this weight range, particularly for children under 40 kg).

The exact dosage will be determined by the practitioner, in light of factors related to the subject. Dosage and administration may be adjusted to provide sufficient levels of the active agent(s) or to maintain the desired effect. Administration factors that may be taken into account include the severity of the disease state, general health of the subject, ethnicity, age, weight, and gender of the subject, diet, time and frequency of administration, drug combination(s), reaction sensitivities, tolerance for toxicity related to drug metabolites, experience with other cancer therapies and regimens, and tolerance/response to such therapies and regimens.

### COMBINATION THERAPIES

The methods of treating cancer by administering an ASD form of Compound A or a pharmaceutical composition thereof disclosed above can further comprise administering to a subject in need thereof an effective amount of an ASD form of Compound A or a pharmaceutical composition thereof in combination with one or more additional agents selected from anti-cancer agents, anti-proliferative agents, chemotherapeutic agents, immunomodulatory agents, anti-angiogenic agents, anti-inflammatory agents, an alkylating agents, steroidal and non-steroidal anti-inflammatory agents, pain relievers, leukotriene antagonists, b2-agonists, anticholinergic agents, hormonal agents, biological agents, tubulin binding agents, glucocorticoids, corticosteroid agents, antibacterial agents, antihistamines, anti-malarial agents, anti-viral agents, antibiotics and the like; and, optionally with radiation therapy.

According to the methods described herein, a combination product may include a combination of active ingredients that may be: (1) co-formulated and administered or delivered simultaneously in a combined formulation; (2) delivered sequentially or in parallel as separate formulations; or (3) by any other combination regimen known in the art. When delivered as separate formulations in alternation therapy, the methods described herein may comprise administration or delivery, for example, without limitation, in separate solutions, emulsions, suspensions, tablets, pills or capsules, or by different injections in separate syringes. In general, when administered in alternation, an effective dosage of each active ingredient is administered serially, one dose following another. In contrast, in parallel or simultaneous administration, effective dosages of two or more active ingredients are administered together. Various alternative combinations of intermittent sequential or in parallel combination administration may also be used.

Specific examples of such agents include, but are not limited to, immunomodulatory agents (e.g., interferon, penicillamine and the like), anti-angiogenic agent, anti-inflammatory agents (e.g., adrenocorticoids, corticosteroids (e.g., beclomethasone, budesonide, flunisolide, fluticasone, triamcinolone, methylprednisolone, prednisolone, prednisone, hydrocortisone), glucocorticoids, steroidal and non-steroidal anti- inflammatory drugs (e.g., aspirin, ibuprofen, diclofenac, and COX-2 inhibitors)), pain relievers, leukotriene antagonists (e.g., montelukast, methyl xanthines, zafirlukast, and zileuton), β2-agonists (e.g., albuterol, biterol, fenoterol, isoetharine, metaproterenol, pirbuterol, salbutamol, terbutaline formoterol, salmeterol, and salbutamol terbutaline), anticholinergic agents (e.g., ipratropium bromide and oxitropium bromide), antibacterial agents (e.g., sulfasalazine, dapsone and the like), antihistamines, anti-malarial agents (e.g., hydroxychloroquine), anti-viral agents (e.g., nucleoside analogs (e.g., zidovudine, acyclovir, ganciclovir, vidarabine, idoxuridine, trifluridine, ribavirin, foscarnet, amantadine, rimantadine, saquinavir, indinavir, ritonavir, and AZT) and antibiotics (e.g., dactinomycin (formerly actinomycin), bleomycin, erythromycin, penicillin, mithramycin, and anthramycin (AMC)).

Specific examples of additional agents that may be used in combination with an ASD form of Compound A include, but are not limited to: acivicin; aclarubicin; acodazole hydrochloride; acronine; adozelesin; aldesleukin; altretamine; ambomycin; ametantrone acetate; aminoglutethimide; amsacrine; anastrozole; anthracycline; anthramycin; asparaginase; asperlin; azacitidine; azetepa; azotomycin; batimastat; benzodepa; bicalutamide; bisantrene hydrochloride; bisnafide dimesylate; bisphosphonates (e.g., pamidronate (Aredria^{®}), sodium clondronate (Bonefos^{®}), zoledronic acid (Zometa^{®}), alendronate (Fosamax^{®}), etidronate, ibandornate, cimadronate, risedromate, and tiludromate); bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; demethylation agents; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; EphA2 inhibitors; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; 5-fluorouracil; fluorocitabine; fosquidone; fostriecin sodium; gemcitabine; gemcitabine hydrochloride; histone deacetylase inhibitors; hydroxyurea; idarubicin hydrochloride; ifosfamide; ilmofosine; imatinib mesylate; interleukin II (including recombinant interleukin II, or rIL2), interferon alpha 2a; interferon alpha 2b; interferon alpha n1 ; interferon alpha n3; interferon beta I a; interferon gamma I b; iproplatin; irinotecan hydrochloride; lanreotide acetate; lenalidomide; letrozole; leuprolide acetate; liarozole hydrochloride; lometrexol sodium; lomustine; losoxantrone hydrochloride; masoprocol; maytansine; mechlorethamine hydrochloride; anti-CD2 antibodies; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; volitinib; vorozole; zeniplatin; zinostatin; zorubicin hydrochloride and the like.

Other examples of treating a cancer mediated according to the present method include treatment with an anti-cancer or anti-proliferative agent wherein the anti-cancer or anti-proliferative agent is selected from, but not limited to: 20-Epi-1,25-dihydroxyvitamin D3 (MC 1288, MC 1301, KH 1060); 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti dorsalizing morphogenetic protein-1; antiandrogen, antiestrogen; antineoplaston; antisense oligonucleotides; aphidicolin glycinate; apoptosis gene modulators; apoptosis regulators; apurinic acid; ara-CDP-DL-PTBA (0-palmitoyl-1-thioglycerol); arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole (CaRest M3); CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate (YNK01 or Starasid^{®}); cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; HMG CoA reductase inhibitors (e.g., atorvastatin, cerivastatin, fluvastatin, lescol, lupitor, lovastatin, rosuvastatin, and simvastatin); hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4 iroplact; irsogladine; isobengazole; isohomohalicondrin B; itasetron; jasplakinolide; kahalalide F; lamellarin N triacetate; lanreotide; leinamycin; lenograstim; lentinan sulfate; leptolstatin; letrozole; leukemia inhibiting factor; leukocyte alpha interferon; leuprolide/estrogen/progesterone combinations; leuprorelin; levamisole; LFA-3TIP); liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF tautomerase inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A/myobacterium cell wall skeleton (CWS/MPL); mopidamol; multiple drug resistance gene inhibitor; multiple tumor suppressor 1 based therapy; mustard anticancer agent; mycaperoxide B; mycobacterial cell wall extract; myriaporone; N-acetyldinaline; N-substituted benzamides; nafarelin; nagrestip; naloxone/pentazocine combinations; napavin; naphterpin; nartograstim; nedaplatin; nemorubicin; neridronic acid; neutral endopeptidase; nilutamide; nisamycin; nitric oxide modulators; nitroxide antioxidant; nitrullyn; 06-benzylguanine; octreotide; okicenone; oligonucleotides; onapristone; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine (BCX 34); pentosan polysulfate sodium; pentostatin; pentrozole; perflubron; perfosfamide; perillyl alcohol dehydrogenase; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem cell division inhibitors; stipiamide; stromelysin inhibitors; sulfinosine; superactive vasoactive intestinal peptide antagonist; suradista; suramin; swainsonine; synthetic glycosaminoglycans; tallimustine; 5-fluorouracil; leucovorin; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus derived growth inhibitory factor; urokinase receptor antagonists; vapreotide; variolin B; vector system, erythrocyte gene therapy; thalidomide; velaresol; veramine; verdins; verteporfin; vinorelbine; vinxaltine; volitinib; vorozole; zanoterone; zeniplatin; zilascorb; zinostatin stimalamer and the like.

In some aspects, the additional agent is one or more immunomodulatory agent(s) used in combination with an ASD form of Compound A. Non-limiting examples of immunomodulatory agents include proteinaceous agents such as cytokines, peptide mimetics, and antibodies (e.g., human, humanized, chimeric, monoclonal, polyclonal, Fvs, ScFvs, Fab or F(ab)2 fragments or epitope binding fragments), nucleic acid molecules (e.g., antisense nucleic acid molecules and triple helices), cancer molecules, organic compounds, and inorganic compounds.

In particular, one or more immunomodulatory agents that may be used in combination with an ASD form of Compound A include, but are not limited to, methotrexate, leflunomide, cyclophosphamide, cytoxan, cyclosporine A, minocycline, azathioprine (Imuran^{®}), antibiotics (e.g., FK506 (tacrolimus)), methylprednisolone (MP), corticosteroids, steroids, mycophenolate mofetil, rapamycin (sirolimus), mizoribine, deoxyspergualin, brequinar, malononitriloamindes (e.g., leflunamide), T cell receptor modulators, cytokine receptor modulators, and modulators mast cell modulators.

In one aspect, the immunomodulatory agent is a chemotherapeutic agent. In an alternative aspect, the immunomodulatory agent is an immunomodulatory agent other than a chemotherapeutic agent. In some aspects, the additional agent used described herein is not an immunomodulatory agent.

In some aspects, the additional agent is one or more anti-angiogenic agent(s) that may be used in combination with an ASD form of Compound A. Non-limiting examples of anti-angiogenic agents include proteins, polypeptides, peptides, fusion proteins, antibodies (e.g., human, humanized, chimeric, monoclonal, polyclonal, Fvs, ScFvs, Fab fragments, F(ab)2 fragments, and antigen-binding fragments thereof) such as antibodies that immunospecifically bind to TNF-α, nucleic acid molecules (e.g., antisense molecules or triple helices), organic molecules, inorganic molecules, and cancer molecules that reduce or inhibit angiogenesis. In other aspects, the additional agent described herein is not an anti-angiogenic agent.

In some aspects, the additional agent that may be used in combination with an ASD form of Compound A is one or more anti-inflammatory agent(s). Non-limiting examples of anti-inflammatory agents include any anti-inflammatory agent useful in treating inflammatory disorders. Non-limiting examples of anti-inflammatory agents include non-steroidal anti-inflammatory drugs (NSAIDs), steroidal anti-inflammatory drugs, anticholinergics (e.g., atropine sulfate, atropine methylnitrate, and ipratropium bromide (ATROVENT^{®}), D2-agonists (e.g., albuterol (VENTOLIN^{®} and PROVENTIL^{®}), bitolterol (TORNALATE^{®}), levalbuterol (XOPONEX^{®}), metaproterenol (ALUPENT^{®}), pirbuterol (MAXAIR^{®}), terbutlaine (BRETHAIRE^{®} and BRETHINE^{®}), albuterol (PROVENTIL^{®}, REPETABS^{®}, and VOLMAX^{®}), formoterol (FORADIL AEROLIZER^{®}), salmeterol (SEREVENT^{®} and SEREVENT DISKUS^{®})), methylxanthines (e.g., theophylline (UNIPHYL^{®}, THEO-DUR^{®}, SLO-BID^{®}, AND TEHO-42^{®})) and the like. Examples of NSAIDs include, but are not limited to, aspirin, ibuprofen, celecoxib (CELEBREX^{®}), diclofenac (VOLTAREN^{®}), etodolac (LODINE^{®}), fenoprofen (NALFON^{®}), indomethacin (INDOCIN^{®}), ketoralac (TORADOL^{®}), oxaprozin (DAYPRO^{®}), nabumentone (RELAFEN^{®}), sulindac (CLINORIL^{®}), tolmentin (TOLECTIN^{®}), rofecoxib (VIOXX^{®}), naproxen (ALEVE^{®}, NAPROSYN^{®}), ketoprofen (ACTRON^{®}), nabumetone (RELAFEN^{®}) and the like. Such NSAIDs function by inhibiting a cyclooxgenase enzyme (e.g., COX-1 and/or COX-2). Examples of steroidal anti-inflammatory drugs include, but are not limited to, glucocorticoids, dexamethasone (DECADRON^{®}), corticosteroids (e.g., methylprednisolone (MEDROL^{®})), cortisone, hydrocortisone, prednisone (PREDNISONE^{®} and DELTASONE^{®}), prednisolone (PRELONE^{®} and PEDIAPRED^{®}), triamcinolone, azulfidine, inhibitors of eicosanoids (e.g., prostaglandins, thromboxanes, and leukotrienes) and the like.

In certain aspects, the additional agent that may be used in combination with an ASD form of Compound A is an alkylating agent, a nitrosourea, an antimetabolite, an anthracyclin, a topoisomerase II inhibitor, a mitotic inhibitor and the like. Alkylating agents include, but are not limited to, busulfan, cisplatin, carboplatin, cholormbucil, cyclophosphamide, ifosfamide, decarbazine, mechlorethamine, mephalen, themozolomide and the like. Nitrosoureas include, but are not limited to carmustine (BiCNU^{®}), lomustine (CeeNU^{®}) and the like. Antimetabolites include, but are not limited to, 5-fluorouracil, capecitabine, methotrexate, gemcitabine, cytarabine, fludarabine and the like. Anthracyclins include but are not limited to daunorubicin, doxorubicin, epirubicin, idarubicin, mitoxantrone and the like. Topoisomerase II inhibitors include, but are not limited to, topotecan, irinotecan, etopiside (VP-16), teniposide and the like. Mitotic inhibitors include, but are not limited to taxanes (paclitaxel, docetaxel), and the vinca alkaloids (vinblastine, vincristine, and vinorelbine) and the like.

In more specific aspects, the additional anti-cancer agent, anti-proliferative agent or chemotherapeutic agent that may be used in combination with an ASD form of Compound A includes, and is not limited to aflibercept, amsacrine, bleomycin, busulfan, capecitabine, carboplatin, carmustine, chlorambucil, cisplatin, cladribine, clofarabine, crisantaspase, cyclophosphamide, cytarabine, dacarbazine, dactinomycin, daunorubicin (IV and liposomal), docetaxel, doxorubicin (IV and liposomal), enzastaurin, epirubicin, etoposide, fludarabine, 5-fluorouracil (5-FU), gemcitabine, gliadel implants, hydroxycarbamide, idarubicin, ifosfamide, imatinib mesylate, irinotecan, lanreotide, lenalidomide, leucovorin, lomustine, melphalan, mercaptopurine, mesna, methotrexate, mitomycin, mitoxantrone, octreotide, oxaliplatin, paclitaxel, pemetrexed, pentostatin, procarbazine, raltitrexed, satraplatin, sorafenib, streptozocin, sunitinib, tegafur uracil, temozolomide, teniposide, thalidomide, thiotepa, tioguanine, topotecan, treosulfan, vatalanib, vinblastine, vincristine, vindesine, vinorelbine, volitinib, ZD6474, monoclonal antibodies (such as bevacizumab, cetuximab, IMC-A12, IMC-1121B, medi-522, rituximab and the like), hormonal agents (such as anastrozole, bicalutamide, buserelin, cyproterone, diethylstilbestrol, exemestane, flutamide, goserelin (breast and prostrate), letrozole, leuprorelin, medroxyprogesterone, megestrol acetate, tamoxifen, toremifene, triptorelin and the like), biological agents (such as interferon, interleukin-12 and the like), angiogenesis receptor tyrosine kinase (RTK) inhibitors (such as AE-941, angiostatin, carboxyamidotriazole, cilengitide, endostatin, halofuginone hydrobromide, 2 methoxyestradiol, squalamine lactate, SU6668 and the like), tubulin binding agents (such as combretastatin A4 phosphate and the like), matrix metalloproteinase inhibitors (such as BMS-275291 and the like) and/or serine/threonine/tyrosine kinase inhibitors and an optional nonsteroidal or COX-2 anti inflammatory agents (such as celecoxib and the like) or corticosteroid (such as prednisone and the like).

In more particular aspects, one or more additional anti-cancer, anti-proliferative or chemotherapeutic agents that may be used in combination with an ASD form of Compound A is selected from bevacizumab, carboplatin, cisplatin, docetaxel, doxorubicin, exemestane, gemcitabine, 5-fluorouracil, imatinib, irinotecan, sorafenib, sunitinib, temozolomide, volitinib or combinations thereof.

In some aspects, an ASD form of Compound A and one or more additional anti-cancer, anti-proliferative or chemotherapeutic agents is used in combination with radiation therapy comprising the use of x-rays, gamma rays and other sources of radiation to destroy cancer cells or tumor cells. In specific aspects, the radiation therapy is administered as external beam radiation or teletherapy, wherein the radiation is directed from a remote source. In other aspects, the radiation therapy is administered as internal therapy or brachytherapy wherein a radioactive source is placed close to cancer cells, tumor cells and/or a tumor mass.

Currently available anti-cancer, anti-proliferative or chemotherapeutic agents, their dosage regimens, routes of administration and recommended usage alone or in combination are known in the art and have been described in literature such as the Physician's Desk Reference.

Any anti-cancer, anti-proliferative or chemotherapeutic agent or anti-cancer therapy which is known to be useful, or which has been used or is currently being used for the treatment of a cancer can be used in combination with a pharmaceutical composition comprising an ASD form of Compound A administered as described herein. See, e.g., Gilman et al., Goodman and Gilman's: The Pharmacological Basis of Therapeutics, 10th ed., McGraw-Hill, New York, 2001; The Merck Manual of Diagnosis and Therapy, Berkow, M.D. et al. (eds.), 17th Ed., Merck Sharp & Dohme Research Laboratories, Rahway, NJ, 1999; Cecil Textbook of Medicine, 20th Ed., Bennett and Plum (eds.), W.B. Saunders, Philadelphia, 1996, and Physician's Desk Reference for information regarding cancer therapies (e.g., using prophylactic or therapeutic agents) which have been or are currently being used for preventing, treating and/or managing various types of cancer.

### EXAMPLES

The following examples illustrate aspects of the ASD form of Compound A described herein, as well as illustrating comparative forms of Compound A and compositions thereof. The examples are not to be construed as limitations.

### EXAMPLE 1

Compound A produced according to the process described in WO2014/081906, hereinafter referred to as "Form I" or the "raw" form, was found to be a crystalline white to off-white solid. Based on results from Dynamic Vapor Sorption (DVS), the raw form was found to be somewhat hygroscopic, with approximately 1.3% water absorption at 95% RH, practically insoluble in aqueous phosphate buffer at pH 6.5, with a solubility of <1 µg/mL at about 18 µg/mL in Fasted State Intestinal Fluid (FaSSIF) at pH 6.5. Form I of Compound A has a cLogP of 5.2, indicating that this compound is highly lipophilic and has a weakly basic aromatic amino group (pKa < 3).

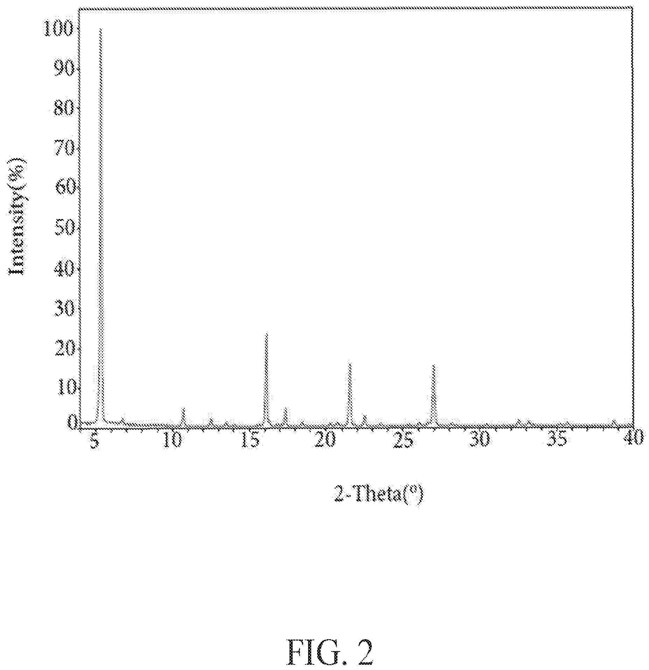

X-ray diffraction analysis of Compound A showed that Form I is crystalline in nature, as shown in FIG. 2. Polymorph screening studies revealed that there are two solvated forms of Compound A, referred to herein as Form A and Form B. Form A was obtained by dissolving Form I of Compound A in ethanol, shaking for 72 hours at 25°C, and centrifuging the resulting mixture to separate solid material from the supernatant, then drying the samples under vacuum overnight at 50°C. The resulting samples are characterized herein by XRPD, DSC, and TGA.

Form B was obtained by dissolving Form I in isopropyl alcohol to form a saturated solution, then the water was added as an anti-solvent while stirring until a precipitate formed. The solid material was separated from the supernatant by centrifugation, dried under vacuum overnight at 50°C, and are characterized herein by XRPD, DSC, and TGA.

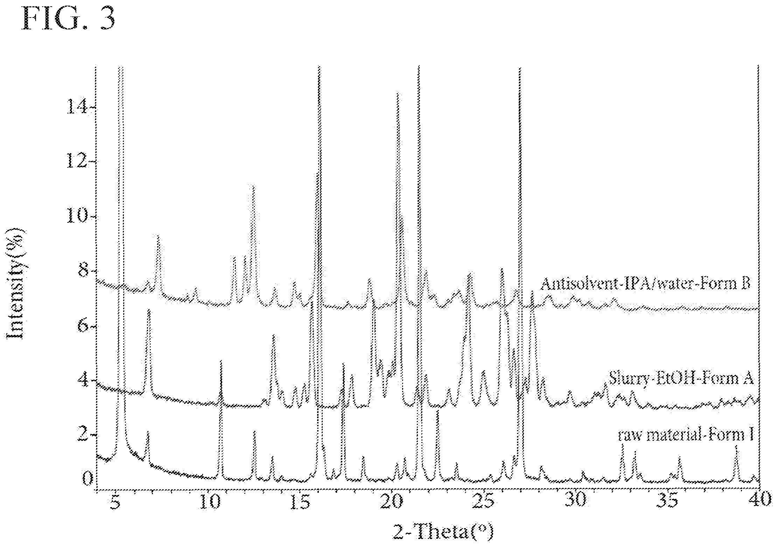

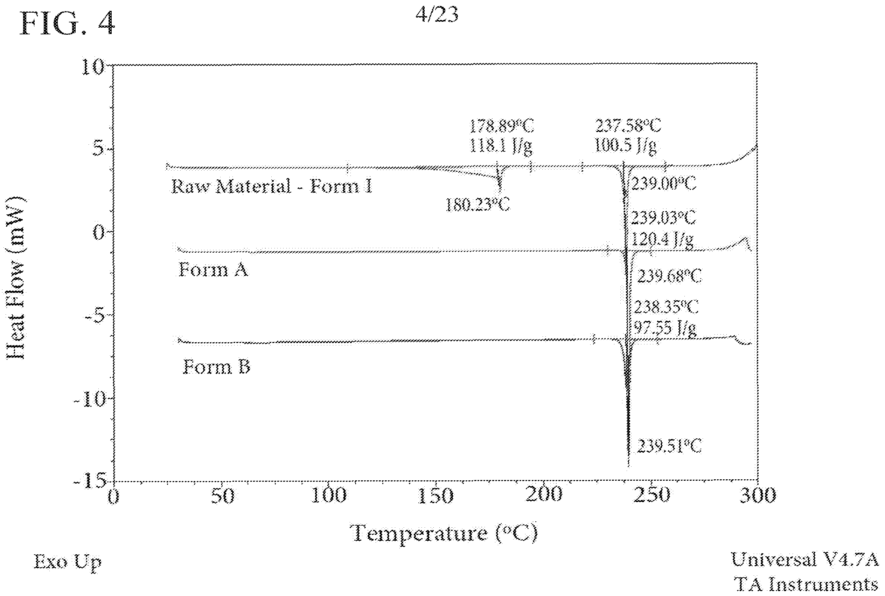

An overlay of the XRPD profiles of Forms I, A, and B are shown in FIG. 3. An overlay of the DSC scans of Forms I, A, and B of Compound A are shown in FIG. 4. Polymorph stability studies of the two crystalline polymorphs demonstrated that Form A was more stable than Form B.

### EXAMPLE 2

The presence of surfactants has been found to increase the aqueous solubility of the Form A form of Compound A. Therefore, two solid dosage formulations of Compound A, Form A Tablet 1 and Form A Tablet 2, shown in Table 1, below, were prepared.

**Table 1 - Composition of Two Solid Dosage Forms**

| **Component** | **Form A Tablet 1 (%w/w)** | **Form A Tablet 2 (%w/w)** |
|---|---|---|
| Micronized Compound A | 29.9 | 25.0 |
| Microcrystalline cellulose (Avicel PH102) | 29.8 | 26.9 |
| Mannitol (100SD) | 33.8 | 39.5 |
| Crospovidone CL | 4.0 | 4.0 |
| Poloxamer 407 | 1.0 | -- |
| Sodium Lauryl Sulfate (SLS) | -- | 3.2 |
| Colloidal Silicon Dioxide | 0.5 | 0.5 |
| Magnesium Stearate | 1.0 | 1.0 |

The two tablet dosage forms were prepared as follows. Crystalline Form A of Compound A, sodium lauryl sulfate, and microcrystalline cellulose were pre-mixed, then mannitol, crospovidone, and silica were added and mixed. The admixture was granulated using a dry granulation process and tablets were subsequently obtained using a tablet press. The tablets were crushed and sieved consecutively through 12 and 18 mesh to provide granules. The granules were filled in size 0 or size 00 white opaque gelatin capsules depending on the dose.

The two prototype tablet formulations described above were also evaluated in cymologous monkeys, as described in Example 11, below. The absolute oral bioavailability of the Form A Tablet 1 and 2 were 9% and 34%, respectively, and the AUC₀-inf exposures were 1.77 and 6.98 hr^{∗}µg/mL, respectively. The target exposure (AUC₀-inf) was not achieved with either formulation at the 10 mg/kg dose.

### EXAMPLE 3

Two capsule dosage forms of solubilized crystalline Form A of Compound A were developed, using polyethylene glycol 4000, Tween 80 and two different solvents, Gelucire 44/14 and Solutol HS15, as shown in Table 2, below.

**Table 2 - Composition of two Capsule Formulations**

| **Composition** | **Capsule 1 % w/w** | **Capsule 2 % w/w** |
|---|---|---|
| Compound A | 10 | 10 |
| Polyethylene Glycol 4000 | 50 | 50 |
| Tween 80 | 30 | 30 |
| Gelucire 44/14 | - | 10 |
| Solutol HS15 | 10 | - |

Both capsule dosage forms were evaluated for *in vitro* dissolution behavior in 0.01 N HCl and FaSSIF, pH 6.5. Both showed complete dissolution in 0.01 N HCl and FaSSIF, pH 6.5 in 45 minutes. Both prototype dosage forms were further evaluated in a monkey PK study, described in Example11, below.

### EXAMPLE 4

Two additional capsule dosage forms, with 10 mg and 50 mg doses of crystalline Form A of Compound A, respectively, solubilized in a mixture of polyethylene glycol 4000 and Gelucire 44/14 were also developed for testing in humans. The composition of those two dosage forms is provided in Table 3, below:

**Table 3 - Composition of a Solubilized Capsule Formulation**

| **Component** | **Source** | **Role in the formulation** | **% w/w** | **Capsule Strength** | |
|---|---|---|---|---|---|
| | | | | **10 mg** | **50 mg** |
| Compound A | | Active | 7.0 | 10.00 | 50.00 |
| Polyethylene Glycol 4000, USP-NF, EP | Dow Chemicals | Solubilizer | 50.0 | 71.50 | 357.0 |
| Tween 80, USP-NF, EP | Croda Inc. | Solubilizer, co-surfactant | 30.0 | 18.56 | 92.68 |
| Gelucire 44/14, USP, EP | Gattefosse | Emulsifier, surfactant | 12.98 | 42.90 | 214.2 |
| Butylated hydroxytoluene (BHT) | Spectrum | Anti-oxidant | 0.02 | 0.03 | 0.14 |
| Total | | | 100 | 143 | 714 |

Although the formulation performed well in clinical trials, loading of active pharmaceutical ingredient (API) and the amount of capsules administered was not optimal for treating cancer patients.

### EXAMPLE 5

An ASD form of Compound A was tested as a possible approach to improving the rate of dissolution, solubility, and thereby oral bioavailability. To explore this approach for Compound A, the following polymers were evaluated for preparing amorphous SDDs: Polyvinylpyrrolidone K30 (PVP K30), Polyvinylpyrrolidone vinyl acetate 64 (PVP-VA-64), Polyvinylpyrrolidone standard fine CL (PVP CL), Hypromellose acetate succinate (HPMCAS-L) and Hypromellose phthalate. The individual polymers were triturated with Compound A at 1:1 ratio and analyzed by DSC to examine the thermal behavior of the Compound A-polymer matrix then to select polymers for preparation of the amorphous spray-dried dispersion (SDD).

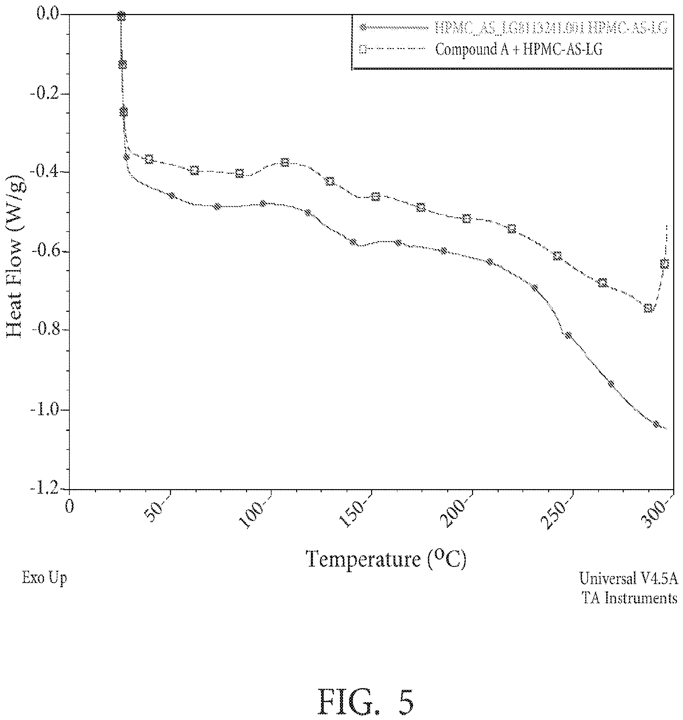

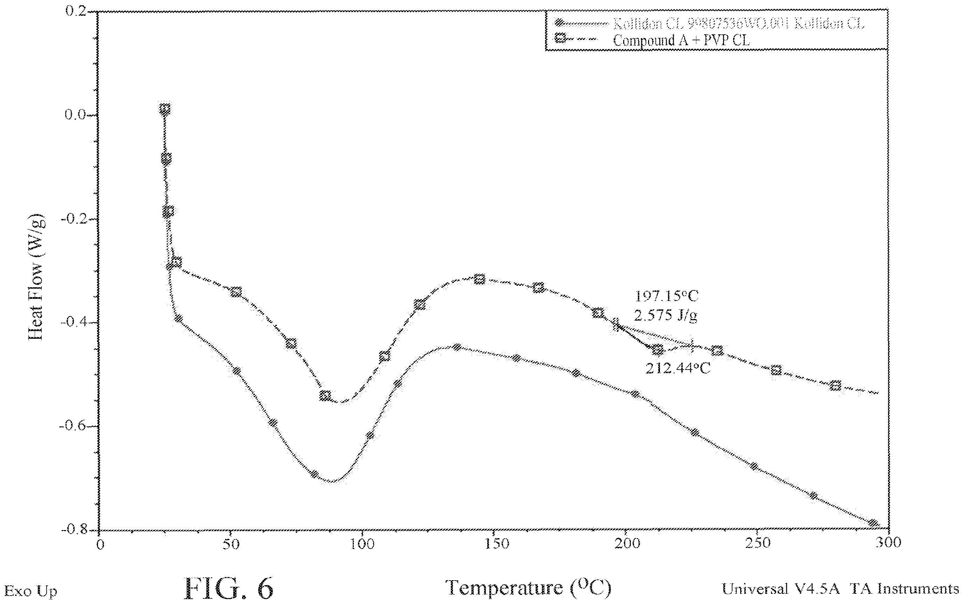

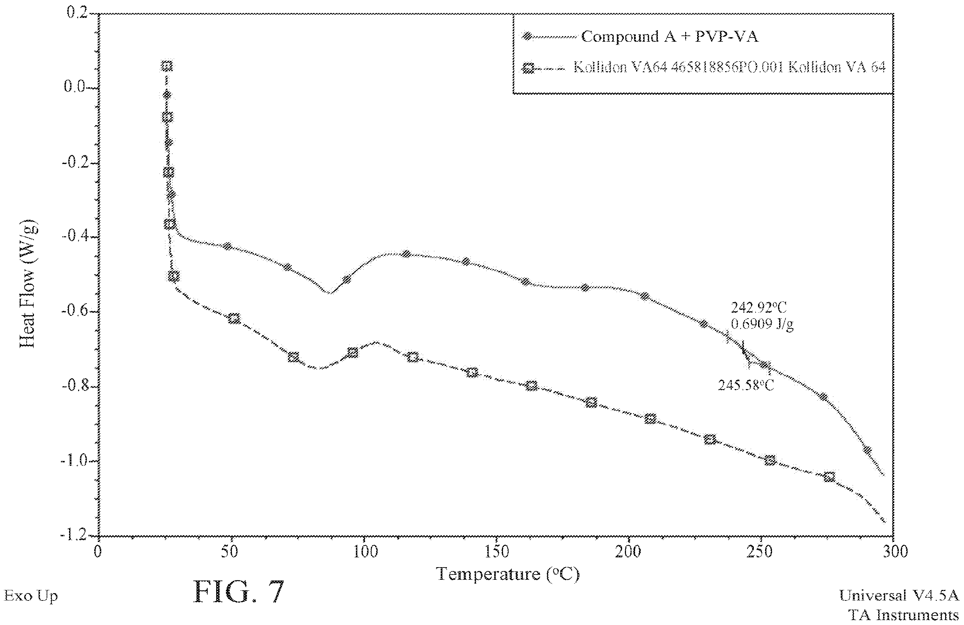

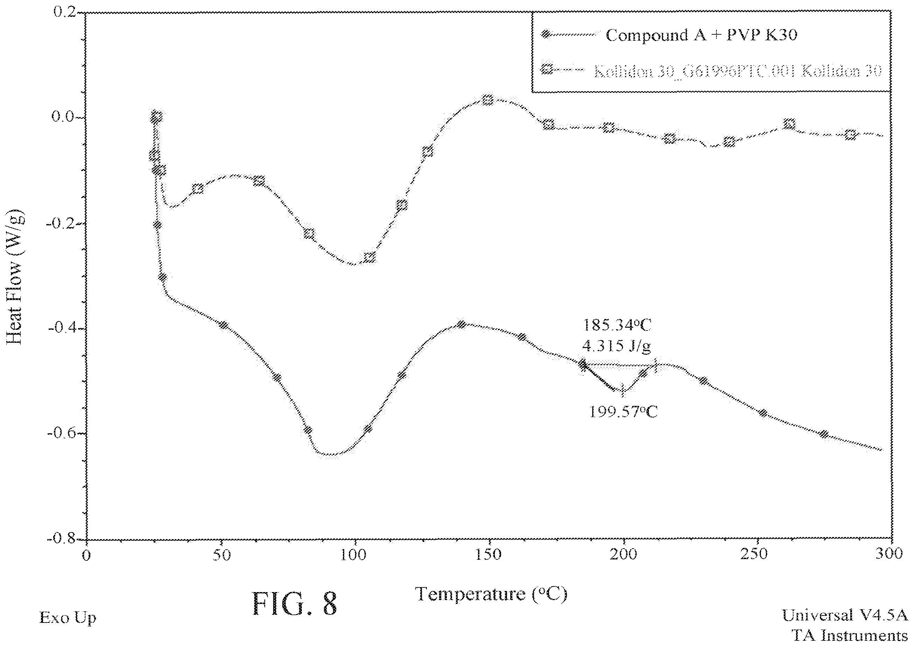

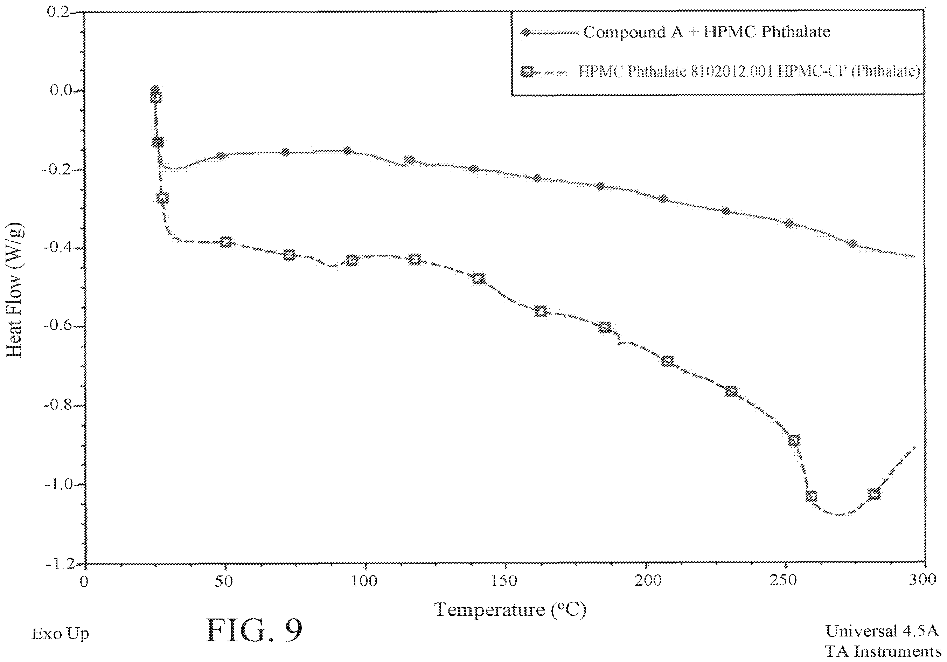

To confirm the solid state of the amorphous form of Compound A in the polymer under molten conditions, thermal characterization was performed using DSC. To confirm the presence or absence of the crystalline Form A of Compound A in the polymer under molten conditions, thermal characterization was performed using DSC. The resulting thermograms are provided in FIGs. 5-9, with the HPMCAS-AS-L thermogram in FIG. 5, the PVP CL thermogram in FIG. 6, the PVP-VA thermogram in FIG. 7, the PVP K30 thermogram in FIG. 8 and the HPMC phthalate thermogram in FIG. 9. In each Figure, thermograms are provided of polymer with and without Compound A. On the basis of the DSC thermograms, wherein the absence of an endothermal peak for the crystalline Form A of Compound A was screened for, two polymers, specifically, HPMC-AS-L, and PVP-VA-64 were chosen for further studies.

### EXAMPLE 6

Different polymers and Compound A-polymer ratios were evaluated using computational and experimental techniques for amorphous physical state. The polymers evaluated were PVP-VA 64, PVP K30, HPMC-AS, Eudragit L100-55, HPMC E3 and HPMC E15. Screening and formulation development incorporated the use of certain assessment tools. Miscibility assessment, an *in silica* simulation, was used to assess phase separation propensity with different stabilizing carriers. The amount of Compound A as an ASD prepared with various process solvents was assessed in various loadings. The results of solvent casting trials with different stabilizing carriers and Compound A loadings was used to further narrow the formulation variables and supersaturation studies. The precipitation inhibition of different stabilizing carriers was also evaluated using a solvent-shift method. Based on polymer miscibility, physicochemical characterization and aqueous solubility measurements, three prototype spray-dried intermediates, [Compound A: HPMC-AS L] and [Compound A: HPMC-AS M] and [Compound A: PVP VA 64] with a ratio of [40: 60 %w/w] were selected for further evaluation. However, PVP VA 64 based SDI was found to be more hygroscopic when compared to both HPMCAS L and HPMCAS M-based formulations.

### EXAMPLE 7

A solvent casting technique was used to obtain an ASD of Compound A. Compound A and polymer were dissolved in methyl ethyl ketone (MEK) and surfactant (sodium lauryl sulphate (SLS) or Poloxamer 407) and water. A surfactant solution was added slowly to the solution of Compound A and polymer (hydroxypropylmethylcellulose acetate succinate (HPMC-AS-L) to obtain a uniform solution. The solvent was evaporated at about 80-85 °C for three days to obtain ASDs of Compound A, as shown in Table 4:

**Table 4: ASD Formulation Compositions**

| **Composition** | **ASD-1 % w/w** | **ASD-2 (P407) % w/w** | **ASD-3 (SLS) % w/w** |
|---|---|---|---|
| Compound A | 50% | 50% | 50% |
| HPMC-AS-LG | 50% | 49% | 49% |
| SLS | - | 1% | - |
| Poloxamer | - | - | 1% |

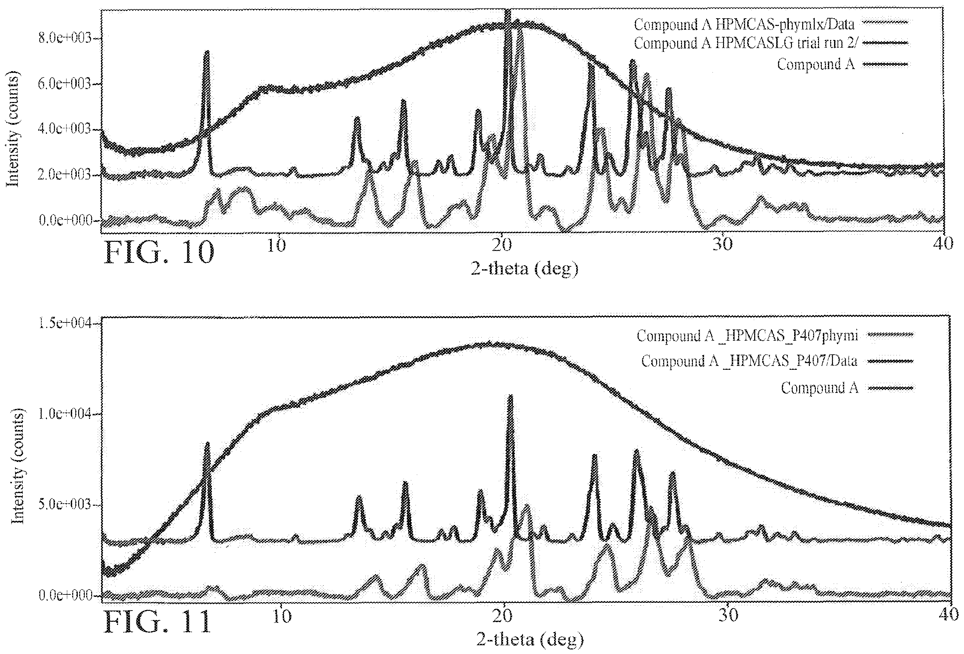

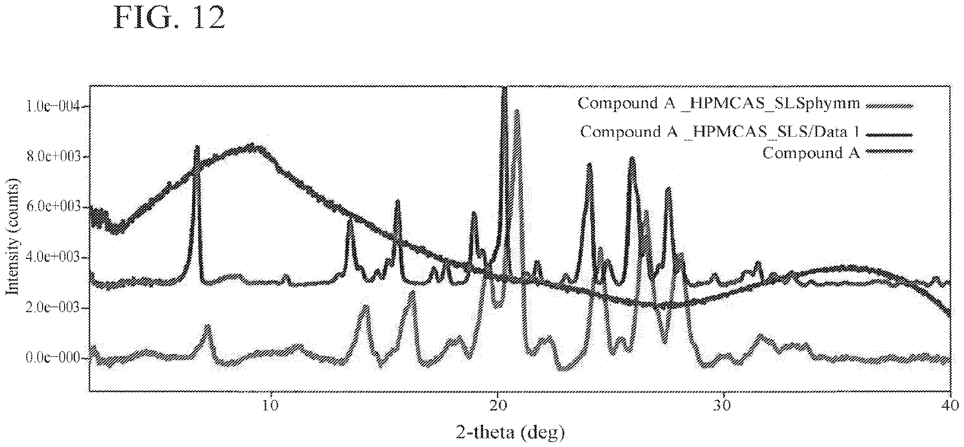

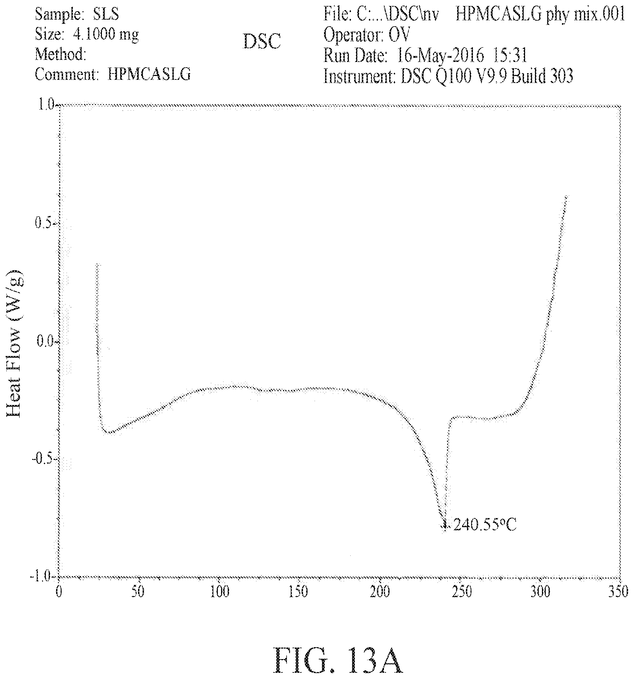

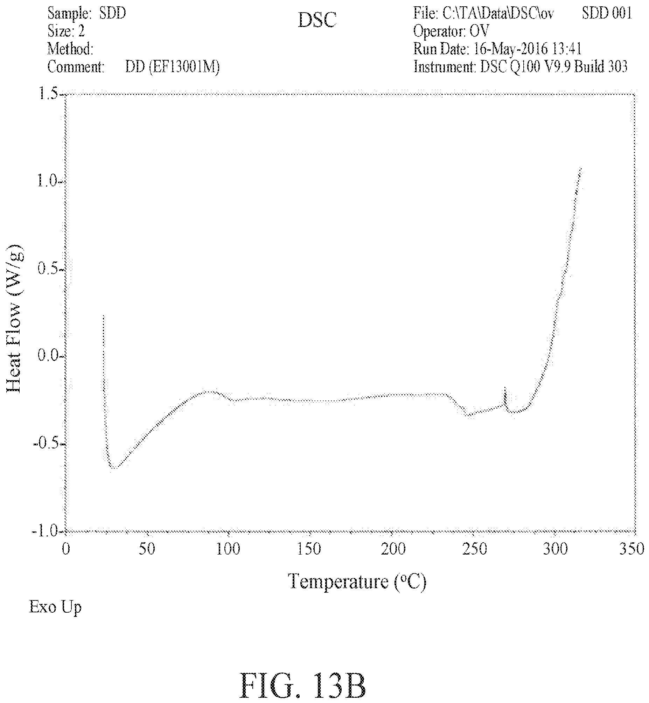

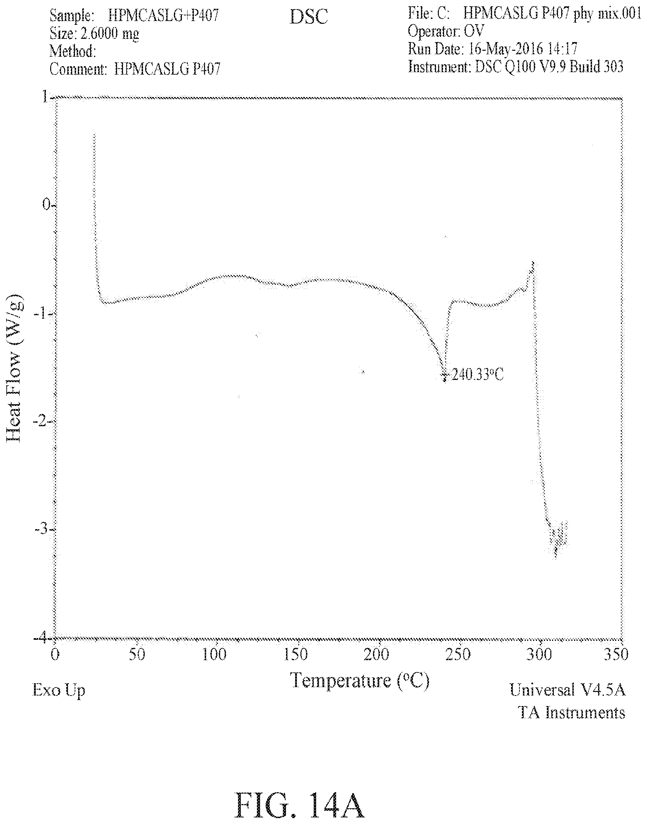

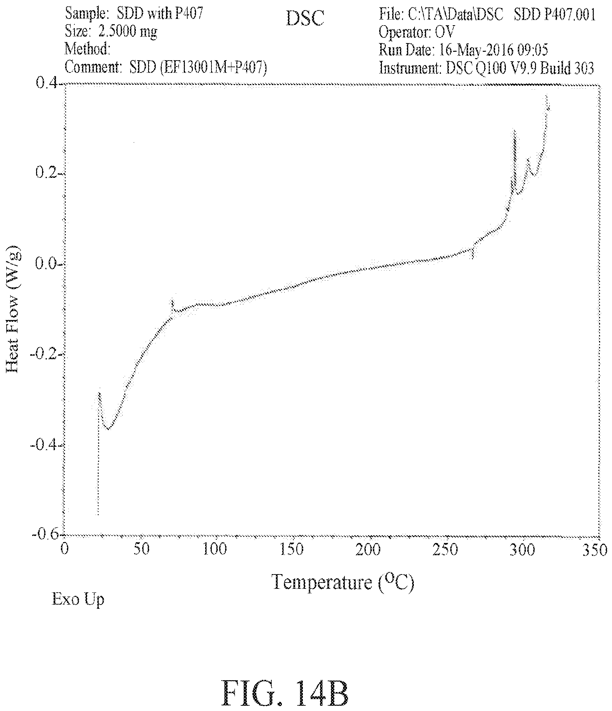

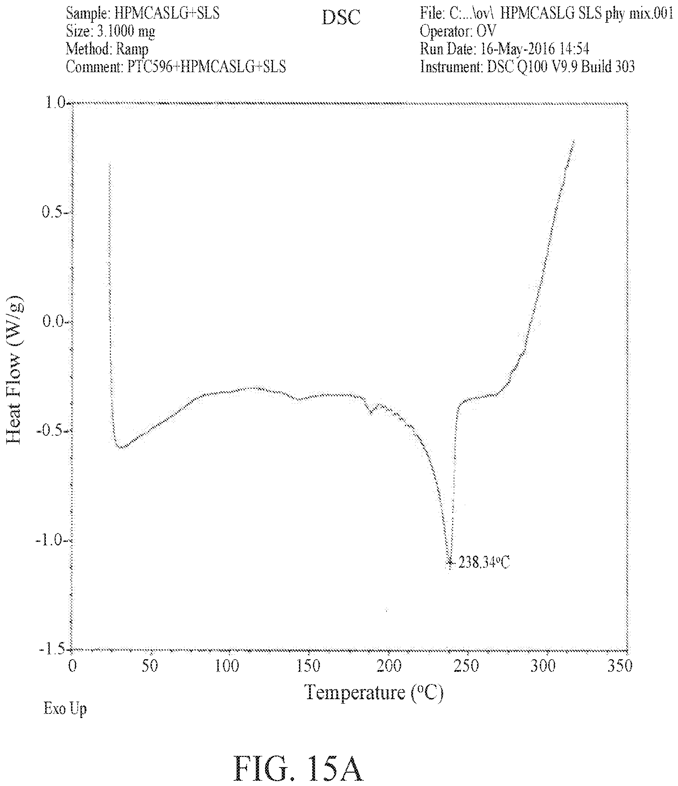

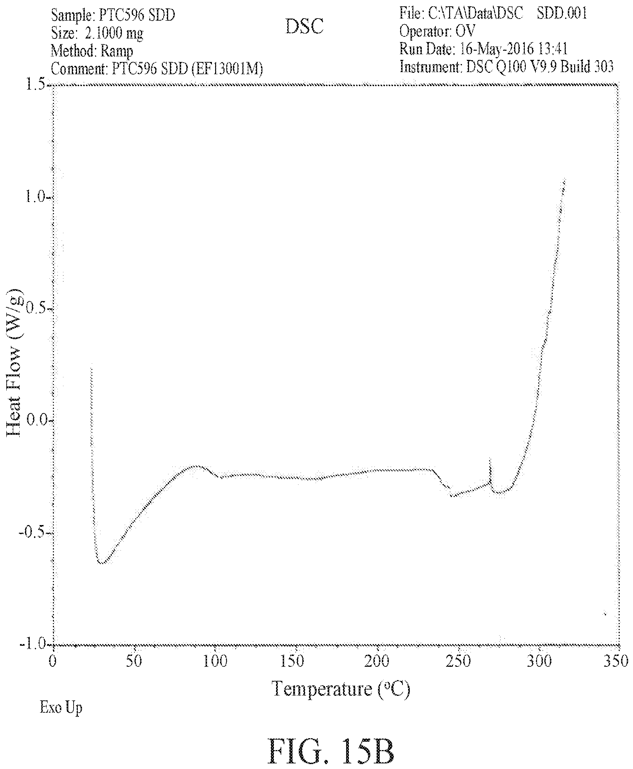

The results of XRPD analysis of ASD-1, ASD-2, and ASD-3 are shown in FIGs 10, 11, and 12, respectively. The DSC results obtained from the mixtures of ASD-1, ASD-2, and ASD-3 prior to solvent casting are shown in FIGs 13A, 14A, and 15A, respectively, while the ASDs produced from the same three mixtures are shown in FIGs 13B, 14B, and 15B, respectively. Both the XRPD and DSC results confirmed that all three mixtures were crystalline prior to solvent casting, with the resulting dispersions produced therefrom found to be amorphous in each case.

Stability studies were conducted on each of the three solid dispersions produced as described above. Samples of each solid dispersion were tested with DSC and XRPD after two weeks at 40°C and 75% relative humidity (RH). The ASD-1 dispersion (no surfactant) and the ASD-3 dispersion (with Poloxamer 407) both remained amorphous, while some peaks appeared in the XRPD of ASD-2 (with SLS), indicating the presence of detectable amounts of crystalline Compound A in ASD-2. The results indicate that a solid dispersion of Compound A can be stable without the presence of SLS as a surfactant in the dispersion.

The solubility of Compound A in solid amorphous dispersions with two different grades of HPMC-AS (L and M grades) was assessed. Compound A was found to be more soluble in amorphous dispersions made with HPMC-AS L compared to those using the HPMC-AS M grade polymer.

### EXAMPLE 8

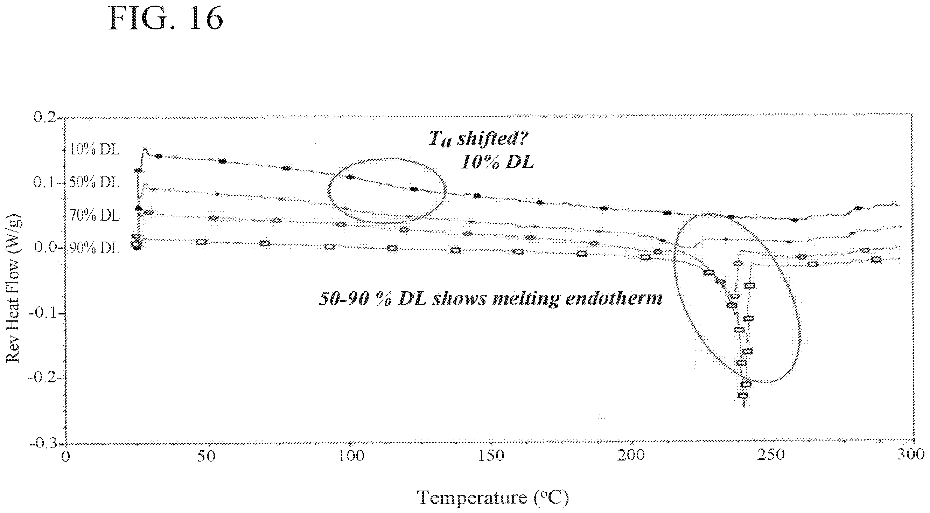

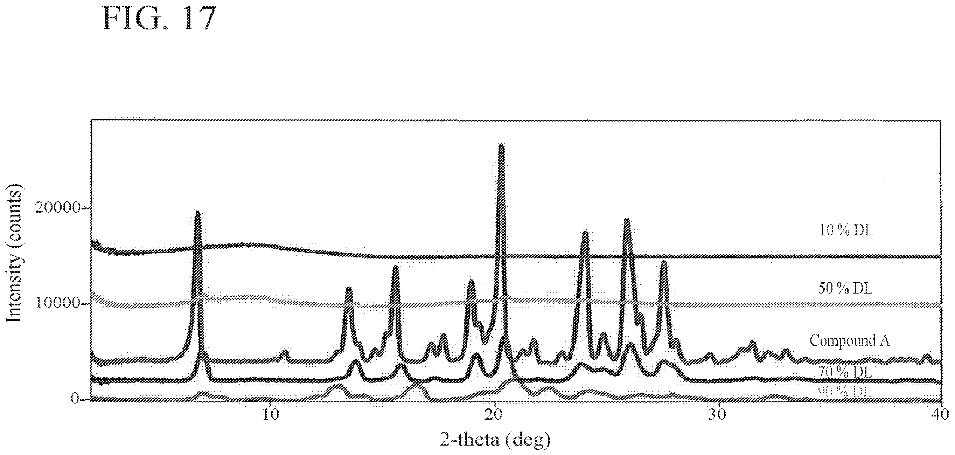

In order to optimize loading of the API, amorphous dispersions of Compound A were prepared by solvent casting at different percentages (10, 50, 70, and 90) of API in the solid dispersion. The 10% and 90% were positive controls for evaluation purposes only. DSC and XRPD scans of the four dispersions are shown in FIGs 16 and 17, respectively. As shown therein, 50% loading of the API in the solid dispersion was not feasible, due to the melting endotherm shown by the DSC of FIG. 16. Accordingly, the loading of the API in the solid dispersion was kept at 40%.

### EXAMPLE 9

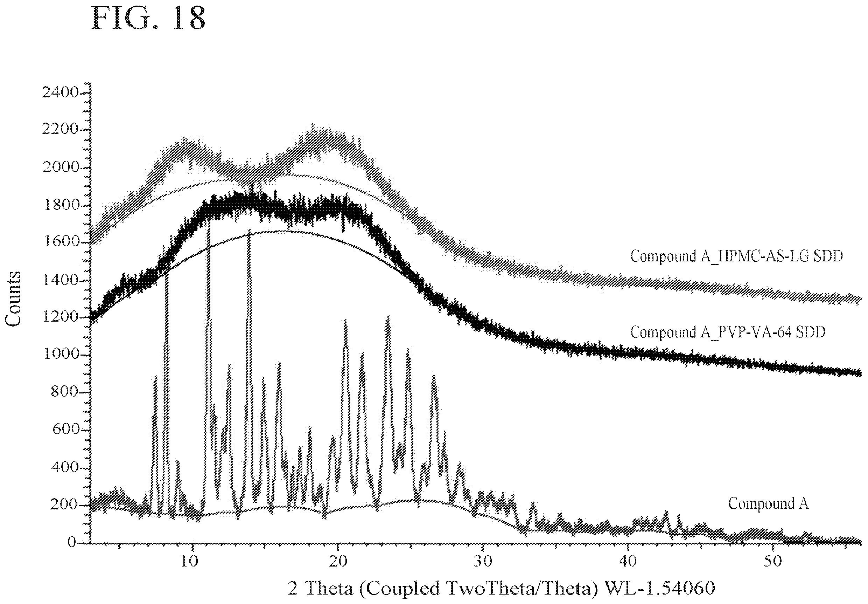

Spray dried dispersions (SDDs) were prepared with each of the two stabilizing polymers identified for further study in Example 5, above, HPMC-AS-LG and PVP-VA-64 by dissolving each polymer and Compound A completely in acetone before spray drying. Powder X-ray diffraction results obtained from each SDD and from Compound A alone are shown in FIG. 18. The XRPD results showed Compound A in both dispersions was amorphous.

### EXAMPLE 10

Formulations of SDDs of Compound A and either HPMC-AS-L (SDD1) or PVP-VA-64 (SDD2) were prepared according to Table 5, below:

**Table 5 - Spray Dry Dispersion Formulations**

| **Component** | **SDD1 % w/w** | **SDD2 % w/w** |
|---|---|---|
| Compound A Spray Dried Dispersion (SDD) (PVP-VA-64) (25%w/w loading) | 70 | - |
| Compound A Spray Dried Dispersion (SDD) (HPMC-AS-LG) (25%w/w loading) | - | 70 |
| Microcrystalline cellulose (Avicel PH102) | 20 | 20 |
| Mannitol (100SD) | 5 | 5 |
| Crospovidone CL | 4 | 4 |
| Colloidal Silicon Dioxide | 0.5 | 0.5 |
| Magnesium Stearate | 0.5 | 0.5 |

The two SDDs were prepared in the same way as described in Example 8, above. Compound A SDDs, SLS and microcrystalline cellulose were pre-mixed in a low shear blender. Mannitol, crospovidone, and silica were added and mixed in a low shear blender. The dry admixture was granulated by dry granulation process by preparing slugs on a tablet press. The slugs were crushed and sieved consecutively through 12 and 18 mesh to provide granules and the granules with the final API loading of 17% w/w were filled in size 0 white opaque gelatin capsules (50 mg strength).

The two formulations, SDD1 and SDD2 were evaluated for dissolution behavior in biorelevant media, Fasted State Simulated Intestinal Fluid (FaSSIF), pH 6.5, using the dissolution parameters shown in Table 6, below:

**Table 6 - Dissolution Parameters**

| | |
|---|---|
| Dissolution Apparatus | USP Apparatus II (Paddles) |
| Temperature | 37°C ± 0.5°C |
| Rotation Speed | 50 rpm |
| Dissolution Buffer/Volume¹ | 600 mL; FaSSIF, pH 6.5 |
| No. of capsules | One size 0 gelatin capsules |
| Strength | 50 mg |
| Sampling Volume² | 5 mL |
| Sampling Time points (Tₘᵢₙ) | (T₀ ,T₅, T₁₅, T₃₀, T₄₅,T₆₀, T₁₂₀ ) |

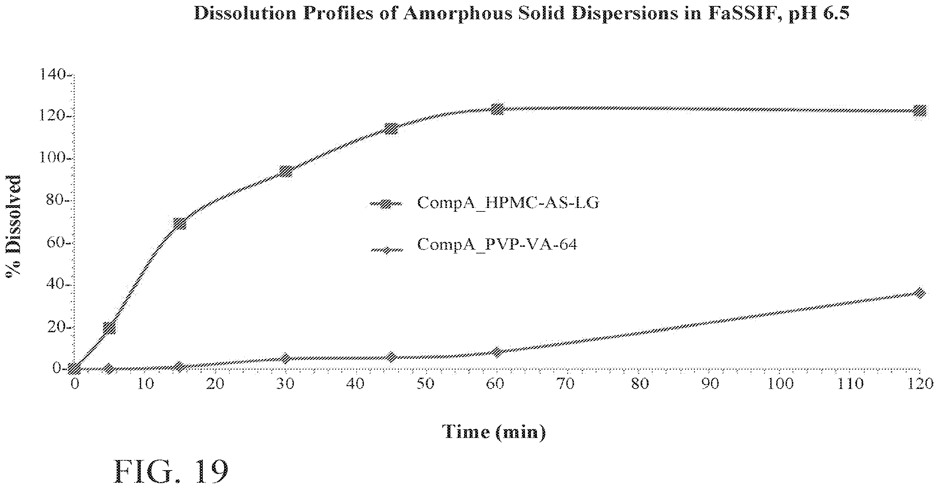

The resulting dissolution profiles for the two SDD formulations are shown in FIG. 19. The dissolution profiles for the SDD made from HPMC-AS-L showed complete dissolution in 45 minutes.

### EXAMPLE 11

Pharmacokinetic monkey studies were conducted, comparing the two tablets of crystalline Compound A produced in Example 2, the capsule dosage forms produced in Example 3, and the SDD2 capsules produced in Example 8, above and a prototype formulation of a spray dried intermediate (SDI) with the composition shown in Table 7, below:

**Table 7 - SDI Tablet Composition for Monkey PK Studies**

| **Component** | **% w/w** |
|---|---|
| Compound A SDI | 47.6 |
| [Compound A: HPMC-AS L] | |
| [Compound A: HPMC-AS M], 40:60 %w/w | |
| Microcrystalline cellulose, NF | 23.8 |
| Lactose monohydrate, NF | 17.1 |
| Croscarmellose sodium | 4.8 |
| Colloidal silicon dioxide, NF | 0.95 |
| Magnesium stearate, NF | 0.95 |
| Poloxamer 407, NF | 4.8 (10% w/w of SDI) |
| Total | 100 |

The results of the study are summarized in Table 8, below:

**Table 8 - Monkey PK Study Results**

| **Formulation** | **Detail** | **Exposure µg^{∗}hr/mL** | **Absolute Oral Bioavailability (F %)** |
|---|---|---|---|
| Capsule 1 (Example 3) | Solutol HS15, 7 % loading | 15 | 74 |
| Capsule 2 (Example 3) | Gelucire 44/14, 7 % loading | 26 | 126 |
| Form A Tablet 1 (Example 2) | Poloxamer 407 as surfactant | 6.9 | 34 |
| Form A Tablet 2 (Example 2) | Sodium Lauryl Sulfate as surfactant | 1.8 | 9 |
| Amorphous SDD1 (Example 6) | HPMC-AS as the polymer | 14 | 70 |

As can be seen from Table 8, at a dose of about 50 mg/animal (approximately 10 mg/kg), the average systemic exposure (AUC_{0-inf}) of Compound A when administered in the solubilized formulation of Capsule 2 (7% API loading) provided the highest comparative bioavailability and exposure. The amorphous SDD1 formulation showed comparably good exposure (AUC: 14433 ng*hr/mL) and an absolute bioavailability of about 70%.

A formulation with a Compound A and HPMC-AS LG SDD with 40% loading was developed. An excipient compatibility study identified certain excipients found to be stable with an ASD form of Compound A. Excipients selected from microcrystalline cellulose, lactose monohydrate, croscarmellose sodium, Poloxamer 407, colloidal silicon dioxide and the SDD intermediate (SDI) form of Compound A were premixed and sieved. Magnesium stearate was subsequently added as a lubricant to the premixture. The density of the premixture was increased using a dry granulation process. The lubricant magnesium stearate and the extragranular excipients microcrystalline cellulose, lactose monohydrate and croscarmellose sodium were subsequently added to the granulate admixture. The admixture having the composition shown in Table 9 below was compressed into tablets of different strengths.

**Table 9 - Admixture Composition**

| **Ingredient Name** | **%w/w** | **g/batch** |
|---|---|---|
| Compound A/HPMC-AS (40%) SDD | 50 | 2000 |
| Microcrystalline cellulose | 22.6 | 903 |
| Lactose monohydrate | 15.9 | 635 |
| Croscarmellose sodium | 4.7 | 188 |
| Poloxamer 407 | 5.0 | 200 |
| Colloidal silicon dioxide | 0.90 | 36 |
| Magnesium stearate | 0.95 | 38 |
| TOTAL: | 100.0 | 4000 |

For comparison purposes, a placebo tablet with the same excipients as the admixture was prepared, absent the SDD.

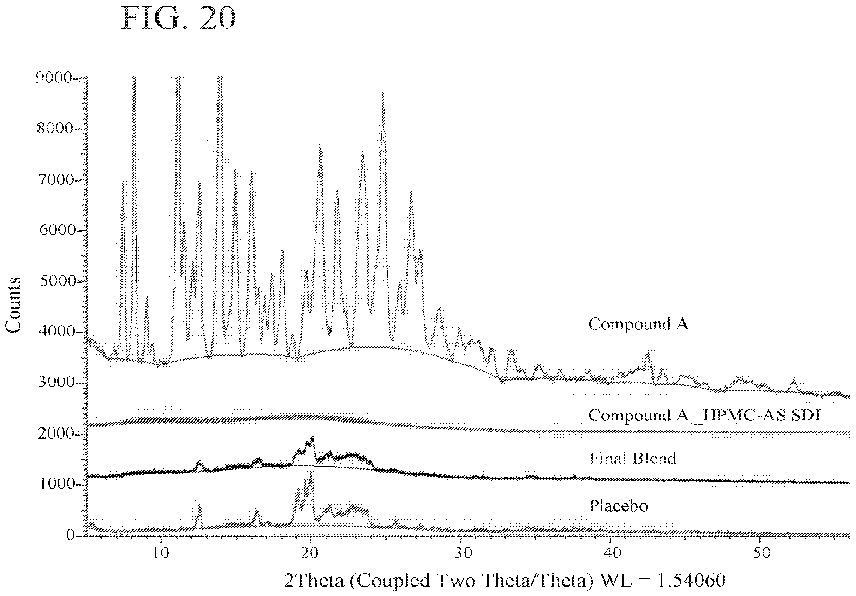

An admixture having Form A of Compound A, the Compound A/HPMC-AS (40%) SDD and placebo were each analyzed by X-ray powder diffractometry. The resulting XRPDs are shown in FIG. 20. The results demonstrated that the SDD and SDD admixture were amorphous. The only peaks in the admixture XRPD were from the excipients, as can be seen in comparison with the placebo XRPD.

### EXAMPLE 12

Tablets having 50 mg and 200 mg of an SDD form of Compound A were produced from a single granulate batch, using different amounts of the granulate for each of the two dosage strengths. The composition of the granulate for each of the two tablet dosages is shown in Table 10, below:

**Table 10 - Composition of 50 and 200 mg Strength Compound A SDD Tablets**

| Ingredient name | **% (w/w)** | **mg/unit** | |
|---|---|---|---|
| | | 50 mg Strength | 200 mg Strength |
| Compound A (40%)/HPMC-AS SDD | 50.0 | 125.0 | 500.0 |
| Microcrystalline cellulose | 22.6 | 56.45 | 225.8 |
| Lactose monohydrate | 15.9 | 39.68 | 158.7 |
| Croscarmellose sodium | 4.7 | 11.75 | 47.0 |
| Colloidal silicon dioxide | 0.9 | 2.25 | 9.0 |
| Poloxamer 407 | 5.0 | 12.50 | 50.0 |
| Magnesium stearate | 0.95 | 2.38 | 9.5 |
| **Total** | **100** | **250** | **1000** |

Granules were obtained through dry granulation, milling and mixing process. A dry granulation was obtained by sieving together a mixture of microcrystalline cellulose, lactose monohydrate, croscarmellose sodium, Poloxamer 407, colloidal silicon dioxide and the SDD, then adding magnesium stearate and mixing to obtain a first admixture. The density of the first admixture was increased by dry granulation to provide a first portion of granules. A second portion of magnesium stearate was sieved with a 30-mesh sieve, then combined with the first portion of granules and mixed in a V-blender for 2 minutes to obtain a second admixture. The second portion of granules were then obtained using a roller compactor and milled. The milled granules were added to the V-blender and mixed with the extragranular excipient's microcrystalline cellulose, lactose monohydrate and croscarmellose sodium. The extragranular excipients were sieved through a 30-mesh sieve, then added to the V-blender and mixed with the granules for 2 minutes to provide a third admixture. The third admixture was compressed into different tablet strengths using die that provided conforming tablet sizes. Both tablet dosage forms were found to be stable at 25°C and 60% relative humidity during a 12 month stability study.

The 50 mg and 200 mg tablets produced as described in this Example have improved dissolution and bioavailability while providing ease of dosing with a reduced amount of dosage form to be administered and increased API loading, where 200 mg tablets given twice per day or 50 mg tablets given four times per day will provide the therapeutically effective amount to be used to treat human patients with various forms of cancer, including diffuse intrinsic pontine glioblastoma (DIPG), ovarian cancer, pancreatic cancer, sarcomas such as leiomyosarcoma, and hematologic cancers such as acute myeloid leukemia.

### EXAMPLE 13

The 50 mg and 200 mg tablets produced as described in Example 12, above, were suspended in water or apple juice, and each resulting suspension was tested for stability at room temperature over 24 hours. Each tablet disintegrated in less than 3 minutes. XRPD and HPLC showed that Compound A remained amorphous in each suspension, with no impurities detected.

Having fully described the subject matter of the claims, it will be understood by those having ordinary skill in the art that the same can be performed within a wide range of equivalents without affecting the scope of the subject matter or aspects described herein.

### EXAMPLE 14

Active formulation tablets having the composition as described in Table 10 were tested for stability under different storage conditions. At 25°C/60% relative humidity (RH), both 50mg and 200mg Compound A tablets demonstrated acceptable stability for 24 months. However, at 40°C / 75% relative humidity (RH) at 6 months, both 50 mg and 200 mg tablets failed to disintegrate. This incomplete dissolution was pH independent and X-ray Powder Diffraction (XRPD) and mDSC analyses confirmed Compound A was both chemically and physically stable in tablet form and remained in an amorphous state even after a year at 40°C / 75% relative humidity (RH).

To determine if lowering the concentration of Poloxamer 407 (5% w/w) in the active tablet formulation could mitigate the observed dissolution slowdown, 25 mg strength tablets were prepared with varying concentrations of Poloxamer 407 (1% w/w, 2% w/w or 5% w/w; Table 11) and placed at 5°C (Control), 25°C/60% RH under closed conditions or 40°C/75% RH under open and closed conditions. The hardness of all tablets tested was maintained at an average of 4.3 kP.

**Table 11: Composition of Active Tablet Formulations with Varying Concentrations of Poloxamer 407**

| | **0% P407** | **1% P407** | **2% P407** | **5% P407 & 2% SiO2** |
|---|---|---|---|---|
| Ingredient | %w /w | %w/w | %w/w | %w/w |
| Compound A: HPMC-AS L SDD (40:60) | 50.00 | 50.00 | 50.00 | 50.00 |
| Macrocrystalline cellulose, USP/NF | 22.58 | 22.58 | 22.58 | 22.58 |
| Lactose monohydrate, USP/NF | 20.87 | 19.87 | 18.87 | 14.77 |
| Croscarmellose sodium, USP/NF | 4.70 | 4.70 | 4.70 | 4.70 |
| Colloidal silicon dioxide, USP/NF | 0.90 | 0.90 | 0.90 | 200 |
| Poloxamer 407, USP/NF | 0.00 | 1.00 | 2.00 | 5.00 |
| Magnesium stearate, USP/NF | 0.95 | 0.95 | 0.95 | 0.95 |
| Total | 100.00 | 100.00 | 100.00 | 100.00 |

| | | | | |
|---|---|---|---|---|
| Abbreviations: hydroxypropylmethylcellulose + acetate succinate: HPMCAS; spray-dried dispersion: SDD; USP/NF refers to United States Pharmacopeia (USP) and the National Formulary (NF) respectively. | | | | |

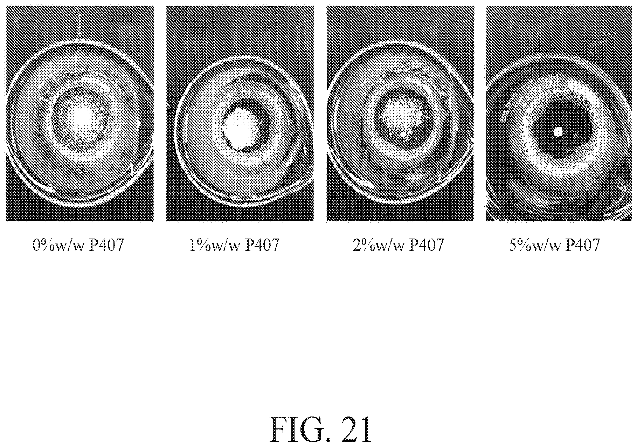

The disintegration and dissolution profiles of the active tablet formulations stored under 40°C/75% RH for 2 weeks under open conditions were assessed in dissolution medium (500 mL of 0.1 N HCl + 0.2% w/v CTAB, 37°C, paddle speed: 75 rpm; see Table 12 and FIG. 21). The active tablet formulation with 5% w/w Poloxamer 407 showed tablet swelling but no disintegration. Consistent with this finding, only minimal API release (-6% release) was measured even after 60 min in the dissolution test. The active tablet formulation with 2% w/w Poloxamer 407 disintegrated into larger chunks, although better than the tablets with 5% w/w Poloxamer 407, the dissolution profile did not attain complete release (-89%) after 60 min. in the dissolution test. However, the active tablet formulation with 1 % w/w Poloxamer 407 readily disintegrated into small particles and the dissolution profile was like active tablet formulations with 0% w/w Poloxamer 407 (- 100% release).

**Table 12: Dissolution Release Profiles of Active Formulations with Varying Concentrations of Poloxamer 407 at 40°C/75% RH under open conditions for 2 weeks**

| | **% Dissolved** | | | | | |
|---|---|---|---|---|---|---|
| | **1% P407** | | **2% P407** | | **5% P407 + 2 % SiO2** | |
| **Time, min** | **Initial*** | **40/75% 2wk** | **Initial*** | **40/75% 2wk** | **Initial*** | **40/75% 2wk** |
| 10 | 69.12 | 53.47 | 66.43 | 46.94 | 66.26 | 1.04 |
| 20 | 84.18 | 77.02 | 83.54 | 68.39 | 87.62 | 2.23 |
| 30 | 91.45 | 84.82 | 89.14 | 78.06 | 91.2 | 3.17 |
| 45 | 95.68 | 93.7 | 92.25 | 87.53 | 96.63 | 4.84 |
| 60 | 97.92 | 97.23 | 95.44 | 89.14 | 96.61 | 6.24 |

| | | | | | | |
|---|---|---|---|---|---|---|
| Initial: refers to the % dissolution of the indicated active tablet formulations (without storage) after 10-60 minutes | | | | | | |

The disintegration and dissolution profiles of these tablet formulations stored at 40°C/75% RH under closed conditions at 6 months were then determined in dissolution medium (500 mL of O.1 N HCl + 0.2% w/v CTAB, 37°C; paddle speed: 75 rpm; see Table 13).

The active tablet formulation with 5% w/w Poloxamer 407 swelled but did not disintegrate and API release at 60 min was minimal (- 6% release) when compared to active tablet formulations with 0% w/w Poloxamer 407 (- 100% release).

The active tablet formulation with 2% w/w Poloxamer 407 disintegrated into larger chunks with almost complete API release at 60 min when compared to active tablet formulations with 0% w/w Poloxamer 407. However, at the 30 min timepoint, there was a - 10% difference in API release when compared to the active tablet formulation with 0% w/w Poloxamer 407.

The active tablet formulation with 1% w/w Poloxamer 407 disintegrated into smaller particles and the dissolution profile showed complete release at 60 min when compared to the active tablet formulation with 0% w/w Poloxamer 407.

**Table 13: Dissolution Release Profiles of Active Formulations with Varying Concentrations of Poloxamer 407 at 40°C/75% RH Closed, 6 months**

| | **Average % Dissolved** | | | |
|---|---|---|---|---|
| **Time Point (min)** | **0% P407** | **1% P407** | **2% P407** | **5% P407** |
| 10 | 70.9 | 63.3 | 52.7 | 1.7 |
| 20 | 88.8 | 82.3 | 71.4 | 2.9 |
| 30 | 95.8 | 91.7 | 82.7 | 3.8 |
| 45 | 101.9 | 98.9 | 92.4 | 5.1 |
| 60 | 103.1 | 100.6 | 96.7 | 6.6 |
| Average tablet weight (mg) | 137.5 | 136.5 | 136.3 | 135.1 |

Varying the concentration of Poloxamer 407 in active tablet formulations had no major impact on Compound A's kinetic solubility or its PK profile and PK parameters when tested in monkeys.

## Claims

1. An amorphous form of 5-fluoro-2-(6-fluoro-2-methyl-1H-benzimidazol-1-yl)-N-[4-(trifluoromethyl)phenyl]pyrimidine-4,6-diamine, having the formula of Compound A:

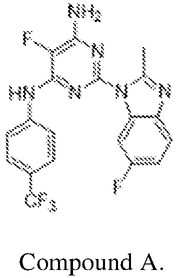

2. The form of claim 1, wherein the amorphous form of Compound A is at least 90% amorphous.

3. The form of claim 2, wherein the amorphous form of Compound A is a substantially pure amorphous form.

4. An amorphous solid dispersion comprising, the amorphous form of any of claims 1, 2 or 3 and a stabilizing polymer.

5. The dispersion of claim 4, wherein the stabilizing polymer is selected from polyvinylpyrrolidone or hydroxypropylmethylcellulose acetate succinate.

6. The dispersion of claim 5, wherein the stabilizing polymer is hydroxypropylmethylcellulose acetate succinate.

7. The dispersion of claim 4, wherein the amount by weight of Compound A to the amount by weight of the stabilizing polymer is in a ratio of between about 5:95 to about 60:40.

8. The dispersion of claim 7, wherein the amount by weight of Compound A to the amount by weight of the stabilizing polymer is in a ratio of between about 10:90 to about 40:60.

9. The dispersion of claim 4, wherein the dispersion is obtained by solvent casting of a solution comprising Compound A and the stabilizing polymer.

10. The dispersion of claim 4, wherein the dispersion is obtained by spray drying a solution comprising Compound A and the stabilizing polymer.

11. A process for preparing the dispersion of claim 4, comprising:
(a) dissolving Compound A and one or more stabilizing polymers in a solvent to form a solution; and,
(b) drying the solution to form the dispersion of claim 4.

12. The process of claim 11, wherein the solvent is selected from acetone, methyl ethyl ketone, dichloromethane, or methanol.

13. The process of claim 11, wherein the solution is dried by solvent casting to form the dispersion of claim 4.

14. The process of claim 11, wherein the solution is dried by spray drying to form the dispersion of claim 4.

15. A pharmaceutical composition comprising, an admixture of the dispersion of claim 4 and one or more pharmaceutically acceptable excipients.

16. The pharmaceutical composition of claim 15, wherein the one or more pharmaceutically acceptable excipients are selected from a diluent, a disintegrant, a lubricant or a surfactant.

17. The pharmaceutical composition of claim 16, wherein the surfactant is selected from sodium lauryl sulfate or Poloxamer 407.

18. A process for preparing the pharmaceutical composition of claim 15 comprising,
(a) grinding the dispersion of claim 4 to form a solid dispersion powder;
(b) admixing the powder with one or more pharmaceutically acceptable excipients to form an admixture;
(c) granulating the admixture to form a granulate; and,
(d) tableting or encapsulating the granulate to form tablets or capsules, respectively.

## Patentansprüche

1. Amorphe Form von 5-Fluor-2-(6-fluor-2-methyl-1H-benzimidazol-1-yl)-N-[4-(trifluormethyl)phenyl]pyrimidin-4,6-diamin, mit der Formel von Verbindung A:

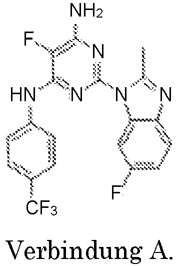

2. Form nach Anspruch 1, wobei die amorphe Form der Verbindung A zu wenigstens 90 % amorph ist.

3. Form nach Anspruch 2, wobei die amorphe Form der Verbindung A eine im Wesentlichen reine amorphe Form ist.

4. Amorphe feste Dispersion, umfassend die amorphe Form nach einem der Ansprüche 1, 2 oder 3 und ein stabilisierendes Polymer.

5. Dispersion nach Anspruch 4, wobei das stabilisierende Polymer ausgewählt ist aus Polyvinylpyrrolidon oder Hydroxypropylmethylcellulose-Acetat-Succinat.

6. Dispersion nach Anspruch 5, wobei das stabilisierende Polymer Hydroxypropylmethylcellulose-Acetat-Succinat ist.

7. Dispersion nach Anspruch 4, wobei die Gewichtsmenge der Verbindung A zur Gewichtsmenge des stabilisierenden Polymers in einem Verhältnis von zwischen ungefähr 5:95 bis ungefähr 60:40 ist.

8. Dispersion nach Anspruch 7, wobei die Gewichtsmenge der Verbindung A zur Gewichtsmenge des stabilisierenden Polymers in einem Verhältnis von zwischen ungefähr 10:90 bis ungefähr 40:60 ist.

9. Dispersion nach Anspruch 4, wobei die Dispersion durch Lösungsmittelgießen einer Lösung, umfassend Verbindung A und das stabilisierende Polymer, erhalten wird.

10. Dispersion nach Anspruch 4, wobei die Dispersion durch Sprühtrocknung einer Lösung, umfassend Verbindung A und das stabilisierende Polymer, erhalten wird.

11. Verfahren zum Herstellen der Dispersion nach Anspruch 4, umfassend:
(a) Auflösen der Verbindung A und eines oder mehrerer stabilisierender Polymere in einem Lösungsmittel, um eine Lösung zu bilden; und,
(b) Trocknen der Lösung, um die Dispersion nach Anspruch 4 zu bilden.

12. Verfahren nach Anspruch 11, wobei das Lösungsmittel ausgewählt ist aus Aceton, Methylethylketon, Dichlormethan, oder Methanol.

13. Verfahren nach Anspruch 11, wobei die Lösung durch Lösungsmittelgießen getrocknet wird, um die Dispersion nach Anspruch 4 zu bilden.

14. Verfahren nach Anspruch 11, wobei die Lösung durch Sprühtrocknung getrocknet wird, um die Dispersion nach Anspruch 4 zu bilden.

15. Pharmazeutische Zusammensetzung, umfassend ein Gemisch der Dispersion nach Anspruch 4 und einen oder mehrere pharmazeutisch verträgliche Hilfsstoffen.

16. Pharmazeutische Zusammensetzung nach Anspruch 15, wobei der eine oder die mehreren pharmazeutisch verträglichen Hilfsstoffe ausgewählt sind aus einem Verdünnungsmittel, einem Sprengmittel, einem Gleitmittel oder einem Tensid.

17. Pharmazeutische Zusammensetzung nach Anspruch 16, wobei das Tensid ausgewählt ist aus Natriumlaurylsulfat oder Poloxamer 407.

18. Verfahren zum Herstellen der pharmazeutischen Zusammensetzung nach Anspruch 15, umfassend,
(a) Mahlen der Dispersion nach Anspruch 4, um ein festes Dispersionspulver zu bilden;
(b) Vermischen des Pulvers mit einem oder mehreren pharmazeutisch verträglichen Hilfsstoffen, um ein Gemisch zu bilden;
(c) Granulieren des Gemischs, um ein Granulat zu bilden; und,
(d) Tablettierung oder Verkapselung des Granulats, um Tabletten bzw. Kapseln zu bilden.

## Revendications

1. Une forme amorphe de 5-fluoro-2-(6-fluoro-2-méthyl-1H-benzimidazol-1-yl)-N-[4-(trifluorométhyl)phényl]pyrimidine-4,6-diamine, ayant la formule du composé A :

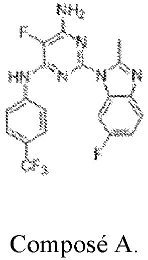

2. Forme selon la revendication 1, dans laquelle la forme amorphe du composé A est au moins à 90 % amorphe.

3. Forme selon la revendication 2, dans laquelle la forme amorphe du composé A est une forme amorphe sensiblement pure.

4. Dispersion solide amorphe comprenant la forme amorphe selon l'une quelconque des revendications 1, 2 ou 3 et un polymère stabilisant.

5. Dispersion selon la revendication 4, dans laquelle le polymère stabilisant est choisi parmi la polyvinylpyrrolidone ou l'acétate succinate d'hydroxypropylméthylcellulose.

6. Dispersion selon la revendication 5, dans laquelle le polymère stabilisant est l'acétate succinate d'hydroxypropylméthylcellulose.

7. Dispersion selon la revendication 4, dans laquelle la quantité en poids de composé A sur la quantité en poids de polymère stabilisant est dans un rapport compris entre environ 5:95 et environ 60:40.

8. Dispersion selon la revendication 7, dans laquelle la quantité en poids de Composé A sur la quantité en poids de polymère stabilisant est dans un rapport compris entre environ 10:90 et environ 40:60.

9. Dispersion selon la revendication 4, dans laquelle la dispersion est obtenue par coulage au solvant d'une solution comprenant le composé A et le polymère stabilisant.

10. Dispersion selon la revendication 4, dans laquelle la dispersion est obtenue par séchage par pulvérisation d'une solution comprenant le composé A et le polymère stabilisant.

11. Procédé de préparation de la dispersion selon la revendication 4, comprenant les étapes consistant à :
(a) dissoudre le composé A et un ou plusieurs polymères stabilisants dans un solvant pour former une solution; et,
(b) sécher la solution pour former la dispersion selon la revendication 4.

12. Procédé selon la revendication 11, dans lequel le solvant est choisi parmi l'acétone, la méthyléthylcétone, le dichlorométhane ou le méthanol.

13. Procédé selon la revendication 11, dans lequel la solution est séchée par coulée au solvant pour former la dispersion selon la revendication 4.

14. Procédé selon la revendication 11, dans lequel la solution est séchée par séchage par pulvérisation pour former la dispersion selon la revendication 4.

15. Composition pharmaceutique comprenant, un mélange de la dispersion selon la revendication 4 et un ou plusieurs excipients pharmaceutiquement acceptables.

16. Composition pharmaceutique selon la revendication 15, dans laquelle le ou les excipients pharmaceutiquement acceptables sont choisis parmi un diluant, un désintégrant, un lubrifiant ou un tensioactif.

17. Composition pharmaceutique selon la revendication 16, dans laquelle le tensioactif est choisi parmi le laurylsulfate de sodium ou le Poloxamer 407.

18. Procédé de préparation de la composition pharmaceutique selon la revendication 15 comprenant les étapes consistant à :
(a) broyer la dispersion selon la revendication 4 pour former une poudre de dispersion solide ;
(b) mélanger la poudre avec un ou plusieurs excipients pharmaceutiquement acceptables pour former un mélange ;
(c) granuler le mélange pour former un granulé ; et,
(d) mettre sous forme de comprimé ou capsule le granulat pour former des comprimés ou des capsules, respectivement.
